(19) Europäisches Patentamt
European Patent Office
Office européen des brevets

(11) Publication number: **0 528 997 B1**

(12) **EUROPEAN PATENT SPECIFICATION**

(45) Date of publication of patent specification: **01.02.95**

(51) Int. Cl.$^6$: **C07C 323/60**, A61K 31/16

(21) Application number: **91911546.9**

(22) Date of filing: **15.05.91**

(86) International application number:
**PCT/US91/03251**

(87) International publication number:
**WO 91/17980 (28.11.91 91/27)**

(54) **DISULFIDE DERIVATIVES OF MERCAPTOACYLAMINO ACIDS.**

(30) Priority: **17.05.90 US 525370**

(43) Date of publication of application:
**03.03.93 Bulletin 93/09**

(45) Publication of the grant of the patent:
**01.02.95 Bulletin 95/05**

(84) Designated Contracting States:
**AT BE CH DE DK ES FR GB GR IT LI LU NL SE**

(56) References cited:
**EP-A- 0 136 883** **EP-A- 0 339 441**
**CH-A- 637 374** **FR-A- 2 556 721**
**US-A- 4 173 704** **US-A- 4 329 495**

**CHEMICAL ABSTRACTS, vol. 97, 19 July 1982, page 339, abstract no. 19653q, Columbus, Ohio, US; M. C. FOURNIE-ZALUSKI et al, "Study of crucial components in enkephalinase inhibitors and synthesis of photoaffinity labels and tritiated derivatives"**

(73) Proprietor: **SCHERING CORPORATION**
**2000 Galloping Hill Road**
**Kenilworth**
**New Jersey 07033 (US)**

(72) Inventor: **HASLANGER, Martin, F.**
**53 W. Glen Avenue**
**Ridgewood, NJ 07450 (US)**
Inventor: **NEUSTADT, Bernard, R.**
**24 Brook Place**
**West Orange, NJ 07052 (US)**
Inventor: **SMITH, Elizabeth, M.**
**166 Grove Avenue**
**Verona, NJ 07044 (US)**

(74) Representative: **von Kreisler, Alek, Dipl.-Chem.**
**Patentanwälte**
**von Kreisler-Selting-Werner**
**Postfach 10 22 41**
**D-50462 Köln (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

CHEMICAL ABSTRACTS, vol. 111, 27 November 1989, page 453, abstract no. 201730x, Columbus, Ohio, US; F. GIMENEZ et al, "Methods for determining thiorphan in aqueous solution. Application to the stability assay of pharmaceutical preparations"

## Description

The present invention relates to disulfide derivatives of mercaptoacylamino acids useful in the treatment of cardiovascular disorders and pain conditions.

Cardiovascular disorders which may be treated with compounds of the present invention include hypertension, congestive heart failure, edema and renal insufficiency.

Human hypertension represents a disease of multiple etiologies. Included among these is a sodium and volume dependent low renin form of hypertension. Drugs that act to control one aspect of hypertension will not necessarily be effective in controlling another.

Enkephalin is a natural opiate receptor agonist which is known to produce a profound analgesia when injected into the brain ventricle of rats. It is also known in the art that enkephalin is acted upon by a group of enzymes known generically as enkephalinases, which are also naturally occurring, and is inactivated thereby.

A variety of mercaptoacylamino acids are known as enkephalinase inhibitors useful as analgesics and in the treatment of hypertension. U.S. 4,774,256 discloses compounds of the formula

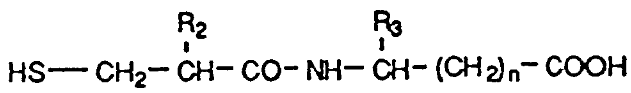

wherein n is 1-15 and $R_2$ and $R_3$ are various aryl, arylalkyl and heteroarylalkyl groups. The compounds are disclosed as having enkephalinase inhibiting activity.

U.S. 4,801,609 to Haslanger et al discloses antihypertensive compounds of the formula

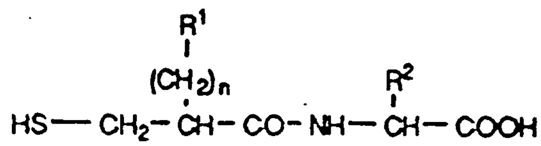

wherein n is 0 or 1; $R^1$ is substituted phenyl and $R^2$ is substituted alkyl, phenyl or heteroaryl. U.S. 4,513,009 to Roques et al disclose similar compounds wherein $R^1$ includes alkyl, optionally substituted phenyl and thienyl; and $R^2$ includes phenyl and substituted alkyl. The compounds are disclosed by Roques et al as principally having enkephalinase inhibitory activity, but are also said to be antihypertensives. U.S. 4,401,677 to Greenberg et al and EPA 38,046 to Wilkinson also disclose compounds of a similar scope, the former disclosing analgesic activity and the latter disclosing a greater specificity for enkephalinase than for angiotensin converting enzyme. U.S. 4,329,495 discloses similar chiral enkephalinase inhibitors wherein $R^2$ is alkylthioalkyl and either $R^1$ is substituted phenyl and the -COOH is replaced by $-CH_2OH$ or $R^1$ is phenyl and the -COOH is present. U.S. 4,500,467 to Kubinyi et al discloses benzoylthio derivatives of compounds similar to those disclosed by Haslanger et al wherein the $R^1$ side chain contains an NH or sulfur atom.

Disulfide derivatives of mercaptoacylamino acids have been identified as angiotensin convering enzyme inhibitors. U.S. 4,053,651 to Ondetti et al discloses disulfide compounds of the formula

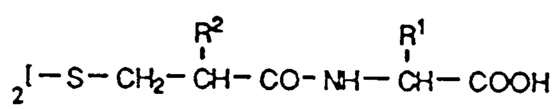

wherein $R^2$ is either hydrogen, lower alkyl or phenyl lower alkyl and $R^1$ includes optionally substituted lower alkyl. U.S. 4,228,007, also to Ondetti et al, discloses similar compounds wherein the $R^2$ side chain contains an oxygen or sulfur atom. Chemical Abstracts, vol. 97, no. 3 (1992) abstract no. 19653q discloses structurally similar compounds wherein the group corresponding to $R^2$ includes dibromobenzyl. U.S. 4,105,776 to Ondetti et al discloses disulfide mercaptoacylproline compounds, and U.S. 4,256,761 to Suh et al discloses disulfide mercapto-acylamino compounds comprising a tertiary amino group.

It is known that the heart secretes a series of peptide hormones called atrial natriuretic factors (ANF) which help to regulate blood pressure, blood volume and the excretion of water, sodium and potassium. ANF were found to produce a short-term reduction in blood pressure and to be useful in the treatment of

congestive heart failure. See P. Needleman et al, "Atriopeptin: A Cardiac Hormone Intimately Involved in Fluid, Electrolyte and Blood-Pressure Homeostasis", N. Engl. J. Med., 314, 13 (1986) pp. 828-834, and M. Cantin et al in "The Heart as an Endocrine Gland", Scientific American, 254 (1986) pg. 7681. U.S. 4,740,499 to Olins discloses a method of prolonging the effect of atrial peptides comprising co-administering thiorphan (a compound within the scope of U.S. 4,513,009) or kelatorphan with an atrial peptide.

A class of drugs known to be effective in treating some types of hypertension is ACE inhibitors, which compounds are useful in blocking the rise in blood pressure caused by increases in vascular resistance and fluid volume due to the formation of angiotensin II from angiotensin I. For a review of ACE inhibitors, see M. Wyvratt and A. Patchett, "Recent Developments in the Design of Angiotensin Converting Enzyme Inhibitors" in Med. Res. Rev. Vol. 5, No. 4 (1985) pp. 483-531.

## SUMMARY OF THE INVENTION

Novel compounds of the present invention are represented by the formulae

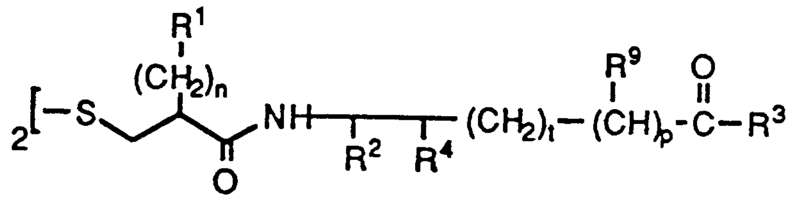

I

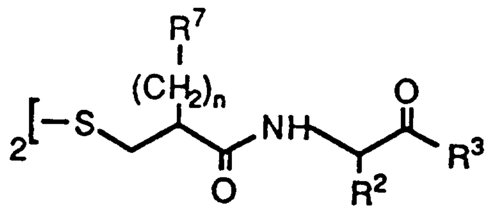

II

wherein

$R^1$ is $C_1$-$C_6$ alkyl, $C_3$-$C_6$ cyclo alkyl, aryl or heteroaryl;

$R^2$ is hydrogen; $C_1$-$C_6$ alkyl; $C_3$-$C_6$ cyclo alkyl; $C_1$-$C_6$ alkyl substituted with hydroxy, $C_1$-$C_6$ alkoxy, mercapto, $C_1$-$C_6$ alkylthio, aryl, heteroaryl, aralkyloxy or aralkylthio; aryl; or heteroaryl;

$R^3$ is $-OR^5$ or $-NR^5R^6$;

$R^4$ and $R^9$ are independently $-(CH_2)_qR^8$;

$R^5$ and $R^6$ are independently selected from the group consisting of hydrogen, $C_1$-$C_6$ alkyl, hydroxy $C_1$-$C_6$ alkyl, $C_1$-$C_6$ alkoxy $C_1$-$C_6$ alkyl and aryl $C_1$-$C_6$ alkyl, or $R^5$ and $R^6$ together with the nitrogen to which they are attached form a 5-7 membered ring;

$R^7$ is phenyl substituted by 1 to 3 substituents selected from the group consisting of $C_1$-$C_6$ alkyl, $C_1$-$C_6$ alkoxy, cycloalkyl, cyano and aminomethyl;

$R^8$ is hydrogen, hydroxy, $C_1$-$C_6$ alkoxy, mercapto, $C_1$-$C_6$ alkylthio, aryl or heteroaryl;

wherein aryl is a mono-cyclic or fused ring bicyclic carbocyclic aromatic group having 6 to 10 ring members and heteroaryl is a mono-cyclic or fused ring bicyclic aromatic group having 5 to 10 ring members wherein 1-2 ring members are independently nitrogen, oxygen or sulfur, and wherein the carbon ring members of the aryl and heteroaryl groups are substituted by 0-3 substituents selected from $C_1$-$C_6$ alkyl, hydroxy, halo, $C_1$-$C_6$ alkoxy, $C_3$-$C_6$ cyclo alkyl, cyano, aminomethyl, trifluoromethyl, phenyl, phenoxy or phenylthio;

n is 1 or 2;

p is 0 or 1;

q is 0, 1 or 2; and

t is 0 or 1;

or a pharmaceutically acceptable salt thereof.

A preferred group of compounds of formula I of the present invention is that wherein t is zero, with compounds wherein p and t are both zero being more preferred. Another group of preferred compounds of formula I is that wherein $R^4$ is hydrogen, hydroxy, methoxy, phenyl or benzyl. Still another preferred group

of compounds of formula I is that wherein $R^2$ is hydrogen or thienyl. Preferred amino acid portions of the compounds of formula I (i.e. the portion $-NH-CH(R^2)-CH(R^4)-(CH_2)_t-(CHR^9)_p-COR^3$) are those wherein p and t are each 0, $R^2$ is hydrogen and $R^4$ is hydroxy or methoxy (e.g. isoserine or O-methyl isoserine); those wherein t is 1, p is 0, $R^2$ is hydrogen and $R^4$ is hydroxy (e.g. homoisoserine); those wherein p and t are each 0, $R^2$ is thienyl and $R^4$ is hydrogen (e.g. $\beta$-thienyl-$\beta$-alanine); and those wherein p and t are each 0, $R^2$ is hydrogen and $R^4$ is phenyl or benzyl.

Other preferred compounds of formula I are those wherein $R^1$ is phenyl or $C_1$-$C_6$ alkyl-substituted phenyl, for example tolyl. Yet another preferred group of compounds is that wherein $R^3$ is hydroxy or $C_1$-$C_6$ alkoxy. A preferred value for n is 1.

Especially preferred compounds of formula I are those wherein $R^1$ is phenyl or tolyl; n is 1; $R^2$ is hydrogen or thienyl; $R^4$ is hydrogen, hydroxy, methoxy, phenyl or benzyl; p is 0; and $R^3$ is hydroxy or lower alkoxy.

A preferred group of compounds of formula II is that wherein $R^7$ is $C_1$-$C_6$ alkyl-substituted phenyl, especially tolyl. Another preferred group of compounds of formula II is that wherein $R^2$ is substituted $C_1$-$C_6$ alkyl, especially $C_1$-$C_6$ alkylthio $C_1$-$C_6$ alkyl, and in particular methylthioethyl. A third group of preferred compounds of formula II is that wherein $R^3$ is hydroxy or $C_1$-$C_6$ alkoxy. A preferred value or n is 1.

Especially preferred compounds of formula II are those wherein $R^7$ is tolyl, $R^2$ is substituted lower alkyl, particularly $C_1$-$C_6$ alkylthio $C_1$-$C_6$ alkyl, and $R^3$ is hydroxy or alkoxy.

The invention also relates to pharmaceutical compositions comprising a mercaptoacylamino acid disulfide derivative of this invention, alone or in combination with an ANF or an ACE inhibitor.

Analgesic pharmaceutical compositions comprising said mercaptoacylamino acid disulfide derivatives are also contemplated.

An additional aspect of the invention relates to a method for preparing the above pharmaceutical compositions, process for the preparation of the compounds of the invention and to their use for the manufacture of a medicament for treating hypertension, congestive heart failure, edema, renal insufficiency, nephrotoxicity or pain.

## DETAILED DESCRIPTION

As used herein, the term "$C_1$-$C_6$ alkyl" means straight or branched alkyl chains of 1 to 6 carbon atoms, and "$C_1$-$C_6$ alkoxy" similarly refers to alkoxy groups having 1 to 6 carbon atoms. $C_3$-$C_6$ Cyclo alkyl means cyclic alkyl groups of 3 to 6 carbon atoms.

"Aryl" means mono-cyclic or fused ring bicyclic carbocyclic aromatic groups having 6 to 10 ring members and "heteroaryl" means mono-cyclic or fused ring bicyclic aromatic groups having 5-10 ring members wherein 1-2 ring members are independently nitrogen, oxygen or sulfur, wherein the carbon ring members of the aryl and heteroaryl groups are substituted by zero to three substituents selected from the group consisting of $C_1$-$C_6$ alkyl, hydroxy, halo, $C_1$-$C_6$ alkoxy, cyclolower alkyl, cyano, aminomethyl, trifluoromethyl, phenyl, phenoxy or phenylthio. Examples of carbocyclic aryl groups are phenyl, $\alpha$-naphthyl and $\beta$-naphthyl, and examples of heterocyclic aryl groups are furyl, thienyl, pyrrolyl, benzofuryl, benzothienyl, indolyl and pyridyl. All positional isomers, e.g. 2-pyridyl, 3-pyridyl, are contemplated.

"Aralkyloxy" and "aralkylthio" refer to aryl $C_1$-$C_6$ alkoxy and aryl $C_1$-$C_6$ alkylthio groups, respectively.

"Halo" refers to fluorine, chlorine, bromine or iodine radicals.

Certain compounds of the invention are acidic e.g., those compounds which possess a carboxyl group. These compounds form pharmaceutically acceptable salts with inorganic and organic bases. Examples of such salts are the sodium, potassium, calcium, aluminum, gold and silver salts. Also included are salts formed with pharmaceutically acceptable amines such as ammonia, alkyl amines, hydroxyalkylamines, and N-methylglucamine.

The salts may be formed by conventional means, as by reacting the free acid form of the product with one or more equivalents of the appropriate base or acid in a solvent or medium in which the salt is insoluble, or in a solvent such as water which is then removed in vacuo or by freeze-drying or by exchanging the cations of an existing salt for another cation on a suitable ion exchange resin.

Compounds of formulae I and II have at least one asymmetrical carbon atom and therefore include various stereoisomers. The invention includes all such isomers both in pure form and in admixture, including racemic mixtures.

An aspect of the present invention described above relates to the combination of a compound of formulae I and II with an ANF. As indicated by Needleman et al., a number of ANF have been isolated so far, all having the same core sequence of 17 amino acids within a cysteine disulfide bridge, but having different N-termini lengths. These peptides represent N-terminal truncated fragments (21-48 amino acids) of

a common preprohormone (151 and 152 amino acids for man and rats, respectively). Human, porcine and bovine carboxy-terminal 28-amino acid peptides are identical and differ from similar peptides in rats and mice in that the former contain a methionine group at position 12 while the latter contain isoleucine. Various synthetic analogs of naturally occuring ANF's also have been found to have comparable biological activity. Examples of ANFs contemplated for use in this invention are α human AP 21 (atriopeptin I), α human AP 28, α human AP 23 (atriopeptin II or APII), α human AP 24, α human AP 25, α human AP 26, α human AP 33, and the corresponding rat sequence of each of the above wherein Met 12 is Ile. See Table I for a comparison of the peptides.

## TABLE I

| HUMAN PEPTIDE | |
|---|---|
| | ‡ (C) * (M) ‡ (C) |
| AP 33 | LAGPRSLRRSSCFGGRMDRIGAQSGLGCNSFRY |
| AP 28 | SLRRSSCFGGRMDRIGAQSGLGCNSFRY |
| AP 26 | RRSSCFGGRMDRIGAQSGLGCNSFRY |
| AP 25 | RSSCFGGRMDRIGAQSGLGCNSFRY |
| AP 24 | SSCFGGRMDRIGAQSGLGCNSFRY |
| AP 23 | SSCFGGRMDRIGAQSGLGCNSFR |
| AP 21 | SSCFGGRMDRIGAQSGLGCNS |

where the amino acids are designated by their single-letter abbreviations, namely

| | | | |
|---|---|---|---|
| A Ala | Alanine | M Met | Methionine |
| C Cys | Cysteine | N Asn | Asparagine |
| D Asp | Aspartic acid | P Pro | Proline |
| F Phe | Phenylalanine | Q Gln | Glutamine |
| G Gly | Glycine | R Arg | Arginine |
| I Ile | Isoleucine | S Ser | Serine |
| L Leu | Leucine | Y Tyr | Tyrosine; |
| M* is replaced by I (Ile), in the rat peptide; and the two C‡ (Cys) residues are connected by a disulfide bridge. | | | |

Another aspect of the invention is the administration of a combination of an ACE inhibitor and a compound of formula I.

Examples of ACE inhibitors are those disclosed in the article by Wyvratt et al., cited above, and in the following U.S. patents: U.S. Patents 4,105,776, 4,468,519, 4,555,506, 4,374,829, 4,462,943, 4,470,973, 4,470,972, 4,350,704, 4,256,761, 4,344,949, 4,508,729, 4,512,924, 4,410,520 and 4,374,847; and the following foreign patents or published patent applications:

British Specification No. 2095682 published October 6, 1982 discloses N-substituted-N-carboxyalkyl aminocarbonyl alkyl glycine derivatives which are said to be angiotensin converting enzyme inhibitors and have the formula

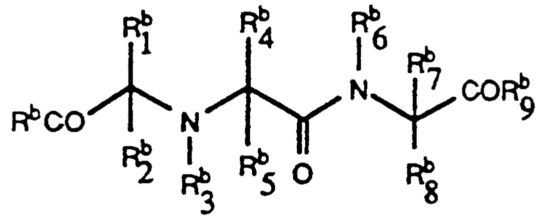

either

(A) $R^b$ and $R_9{}^b$ are OH, 1-6C alkoxy, 2-6C alkenyloxy, di-(1-6C alkyl)amino-(1-6C) alkoxy, 1-6C hydroxyalkoxy, acylamino-(1-6C)alkoxy, acyloxy-(1-6C)alkoxy, aryloxy, aryloxy-(1-6C)alkoxy, $NH_2$, mono- or di-(1-6C alkyl)amino, hydroxyamino or aryl-(1-6C)alkylamino;

$R_1{}^b$-$R_5{}^b$, $R_7{}^b$ and $R_8{}^b$ are 1-20C alkyl, 2-20C alkenyl, 2-20C alkynyl, aryl, aryl-(1-6C) alkyl having 7-12C or heterocyclyl-(1-6C)alkyl having 7-12C;

$R_6{}^b$ is cycloalkyl, polycycloalkyl, partly saturated cycloalkyl or polycycloalkyl, cycloalkyl-(1-6C)alkyl having 3-20C, 6-10C aryl, aryl-(1-6C)alkyl, aryl-(2-6C)alkenyl or aryl-(2-6C) alkynyl; or

$R_2{}^b$ and $R_3{}^b$ together with the C and N atoms to which they are attached or $R_3{}^b$ and $R_5{}^b$ together with the N and C atoms to which they are attached form an N-heterocycle containing 3-5C or 2-4C and a S atom;

all alkyl, alkenyl and alkynyl are optionally substituted by OH, 1-6C alkoxy, thio(sic), 1-6C alkylthio, $NH_2$, mono- or di(1-6C alkyl)amino, halogen or $NO_2$;

all 'cycloalkyl' groups (including poly and partially unsaturated) are optionally substituted by halogen, 1-6C hydroxyalkyl, 1-6C alkoxy, amino-(1-6C alkyl)amino, di-(1-6C alkyl)amino, SH, 1-6C alkylthio, $NO_2$ or $CF_3$; and aryl groups are optionally substituted by OH, 1-6C alkoxy, $NH_2$, mono- or di-(1-6C alkyl) amino, SH, 1-6C alkylthio, 1-6C hydroxyalkyl, 1-6C aminoalkyl, 1-6C thioalkyl, $NO_2$, halogen, $CF_3$, $OCH_2O$, ureido or guanidino; or

(B) $R^b$ and $R_9{}^b$ are H or 1-6C alkoxy;

$R_1{}^b$ and $R_2{}^b$ are H, 1-6C alkyl, aryl-(1-6C) alkyl having 7-12C or heterocyclyl-(1-6C) alkyl having 6-12C;

$R_3{}^b$-$R_5{}^b$, $R_7{}^b$ and $R_8{}^b$ are H or 1-6C alkyl;

$R_6{}^b$ is cycloalkyl, polycycloalkyl, partly saturated cycloalkyl or polycycloalkyl, cycloalkyl-(1-6C) alkyl having 3-20C, aryl or aryl-(1-6C) alkyl; and

aryl has 6-10C and is optionally substituted by 1-6C alkyl, 2-6C alkenyl, 2-6C alkynyl, OH, 1-6C alkoxy, $NH_2$, mono- or di-(1-6C alkyl) amino, SH, 1-6C alkylthio, 1-6C hydroxyalkyl, 1-6C aminoalkyl, 1-6C thioalkyl, $NO_2$, halogen, $CF_3$, $OCH_2O$, ureido or guanidino;

European Patent Application 0 050 800 published May 5, 1982 discloses carboxyalkyl dipeptides derivatives which are said to be angiotensin converting enzyme inhibitors and have the formula

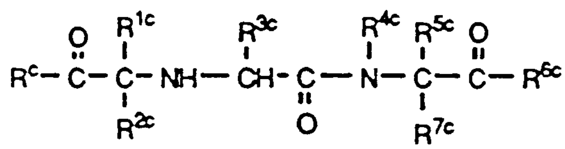

or a pharmaceutically acceptable salt thereof, wherein $R^c$ and $R^{6c}$ are the same or different and are hydroxy, lower alkoxy, lower alkenyloxy, dilower alkylamino lower alkoxy, acylamino lower alkoxy, acyloxy lower alkoxy, aryloxy, aryllower alkoxy, amino, lower alkylamino, dilower alkylamino, hydroxyamino, aryllower alkylamino, or substituted aryloxy or substituted aryllower alkoxy wherein the substituent is methyl, halo or methoxy; $R^{1c}$ is hydrogen, alkyl of from 1 to 10 carbon atoms, substituted lower alkyl wherein the substituent is hydroxy, lower alkoxy, aryloxy, substituted aryloxy, heteroaryloxy, substituted heteroaryloxy, amino, lower alkylamino, diloweralkylamino, acylamino, arylamino, substituted arylamino, guanidino, imidazolyl, indolyl, lower alkylthio, arylthio, substituted arylthio, carboxy, carbamoyl, lower alkoxy carbonyl, aryl, substituted aryl, aralkyloxy, substituted aralkyloxy, aralkylthio or substituted aralkylthio, wherein the aryl or heteroaryl portion of said substituted aryloxy, heteroaryloxy, arylamino, arylthio, aryl, aralkyloxy, aralkylthio group is substituted with a group selected from halo, lower alkyl, hydroxy, lower alkoxy, amino, aminomethyl, carboxyl, cyano, or sulfamoyl; $R^{2c}$ and $R^{7c}$ are the same or different and are hydrogen or lower alkyl; $R^{3c}$ is hydrogen, lower alkyl, phenyl lower alkyl, aminoethylphenyl lower alkyl, hydroxyphenyl lower alkyl, hydroxy lower alkyl, acylamino lower alkyl, amino lower alkyl, dimethylamino lower alkyl,

guanidino lower alkyl, imidazolyl lower alkyl, indolyl lower alkyl, or lower alkyl thio lower alkyl; $R^{4c}$ and $R^{5c}$ are the same or different and are hydrogen, lower alkyl or $Z^c$, or $R^{4c}$ and $R^{5c}$ taken together form a group represented by $Q^c$, $U^c$, $V^c$, $Y^c$, $D^c$ or $E^c$, wherein;

$Z^c$ is

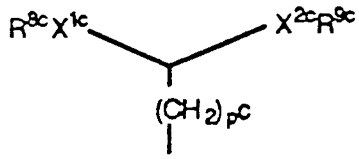

wherein $X^{1c}$ and $X^{2c}$ independent of each other are O, S or $CH_2$, $R^{8c}$ and R9c independent of each other are lower alkyl, lower alkenyl, lower alkynyl, cycloalkyl having 3 to 8 carbon atoms, hydroxy lower alkyl, or $-(CH_2)_n{}^cAr^c$, wherein $n^c$ is 0, 1, 2 or 3 and $Ar^c$ is unsubstituted or substituted phenyl, furyl, thienyl or pyridyl, wherein said substituted phenyl, furyl, thienyl or pyridyl groups are substituted with at least one group that is independently selected from $C_1$ to $C_4$ alkyl, lower alkoxy, lower alkylthio, halo, $CF_3$ and hydroxy, or $R^{8c}$ and $R^{9c}$ taken together form a bridge $W^c$, wherein $W^c$ is a single bond or a methylene bridge or a substituted methylene bridge when at least one of $X^{1c}$ and $X^{2c}$ is methylene, or $W^c$ is an alkylene or substituted alkylene bridge having 2 or 3 carbon atoms, said substituted methylene bridge or said substituted alkylene bridge having one or two substituents selected from lower alkyl, aryl and aryl lower alkyl groups, and $p^c$ is 0, 1 or 2; with the proviso that at least one of $R^{4c}$ and $R^{5c}$ is $Z^c$, with the proviso that if $R^{4c}$ is $Z^c$ and $p^c$ is 0 then $X^{1c}$ and $X^{2c}$ must both be methylene, and with the proviso that if $X^{1c}$ and $X^{2c}$ are both methylene then $R^{8c}$ and $R^{9c}$ must form an alkylene bridge $W^c$;

$Q^c$ is

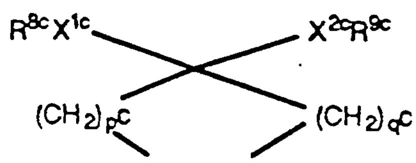

wherein $R^{8c}$, $R^{9c}$, $X^{1c}$ and $X^{2c}$ are as defined above, $p^c$ is 0, 1 or 2, $q^c$ is 0, 1 or 2, with the proviso that the sum of $p^c$ and $q^c$ must be 1, 2 or 3, with the proviso that if $p^c$ is 0 then $X^{1c}$ and $X^{2c}$ must be methylene, and with the proviso that if $X^{1c}$ and $X^{2c}$ are methylene then $R^{8c}$ and $R^{9c}$ taken together form a bridge $W^c$, wherein $W^c$ is as defined above;

$V^c$ is

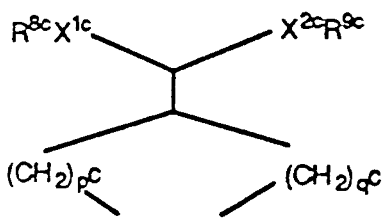

wherein $R^{8c}$, $R^{9c}$, $X^{1c}$ and $X^{2c}$ are as defined above, $p^c$ is 0, 1 or 2 and $q^c$ is 0, 1 or 2, with the proviso that the sum of $p^c$ and $q^c$ is 1, 2 or 3, with the proviso that if $X^{1c}$ and $X^{2c}$ are $CH_2$ then $R^{8c}$ and $R^{9c}$ taken together form a bridge $W^c$, wherein $W^c$ is as defined above;

$U^c$ is

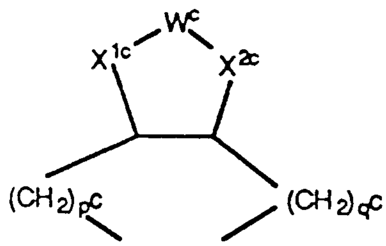

wherein $W^c$ is as defined above (except that $W^c$ may also be a methylene bridge when $X^{1c}$ and $X^{2c}$ are oxygen or sulfur), $X^{1c}$ and $X^{2c}$ are as defined above, $p^c$ is 0, 1 or 2, $q^c$ is 0, 1 or 2, with the proviso that the sum of $p^c$ and $q^c$ is 1 or 2, and with the proviso that if $p^c$ is 0, $X^{1c}$ must be $CH_2$;

$Y^c$ is

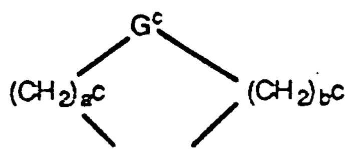

wherein $G^c$ is oxygen, sulfur or $CH_2$, $a^c$ is 2, 3 or 4 and $b^c$ is 1, 2, 3, 4 or 5, with the proviso that the sum of $a^c$ and $b^c$ is 5, 6 or 7 or $G^c$ is $CH_2$, $a^c$ is 0, 1, 2 or 3, $b^c$ is 0, 1, 2 or 3 with the proviso that the sum of $a^c$ and $b^c$ is 1, 2 or 3, with the proviso that the sum of $a^c$ and $b^c$ may be 1, 2 or 3 only if $R^{1c}$ is lower alkyl substituted with aralkylthio or aralkyloxy;

$D^c$ is

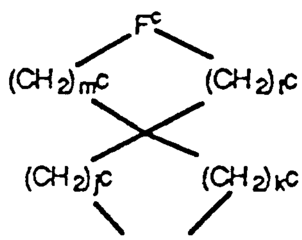

wherein $F^c$ is O or S, $j^c$ is 0, 1 or 2 and $k^c$ is 0, 1 or 2, with the proviso that the sum of $j^c$ and $k^c$ must be 1, 2 or 3, and $m^c$ is 1, 2 or 3 and $t^c$ is 1, 2 or 3, with the proviso that the sum of $m^c$ and $t^c$ must be 2, 3 or 4;

$E^c$ is

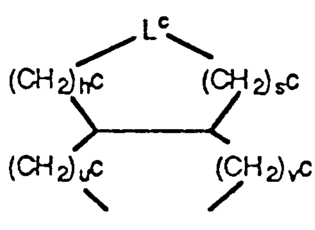

wherein $L^c$ is O or S, $u^c$ is 0, 1 or 2 and $v^c$ is 0, 1 or 2, with the proviso that the sum of $u^c$ and $v^c$ must be 1 or 2, and $h^c$ is 1 or 2 and $s^c$ is 1 or 2, with the proviso that the sum of $h^c$ and $s^c$ must be 2 or 3;

European Patent Application 0 079 522 published May 25, 1983 discloses N-carboxymethyl(amidino) lysyl-proline compounds which are said to be angiotensin converting enzyme inhibitors and have the formula where

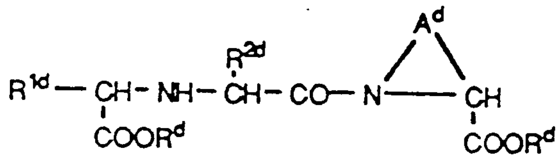

$(I)^d$

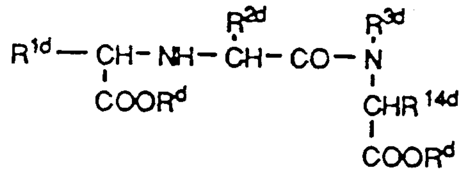

$(Ia)^d$

wherein:

$R^d$ and $R^{2d}$ are independently hydrogen; loweralkyl; aralkyl; or aryl;

$R^{1d}$ is hydrogen; branched or straight chain $C_{1-12}$ alkyl and alkenyl; $C_3$-$C_9$ cycloalkyl and benzofused alkyl; substituted loweralkyl where the substituents are halo, hydroxy loweralkoxy, aryloxy, amino, mono- or diloweralkylamino, acylamino, arylamino, guanidino, mercapto, loweralkylthio, arylthio, carboxy, carboxamido, or loweralkoxycarbonyl; aryl; substituted aryl where the substituents are loweralkyl, loweralkoxy, or halo; arloweralkyl; arloweralkenyl; heteroarloweralkyl; heteroarloweralkenyl; substituted arloweralkyl, substituted arloweralkenyl, substituted heteroarloweralkyl, or substituted heteroarloweralkenyl where the aryl and heteroaryl substituents are halo, dihalo, loweralkyl, hydroxy, loweralkoxy, amino, aminoloweralkyl, acylamino, mono- or diloweralkylamino, carboxyl, haloloweralkyl, nitro, cyano, or sulfonamido, and where the loweralkyl portion of arloweralkyl may be substituted by amino, acylamino, or hydroxyl;

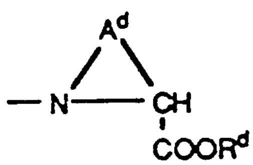

is

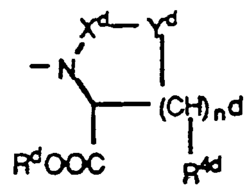

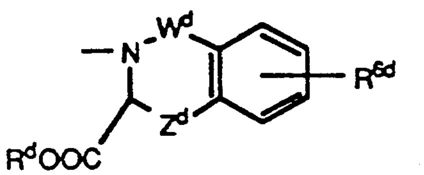

or

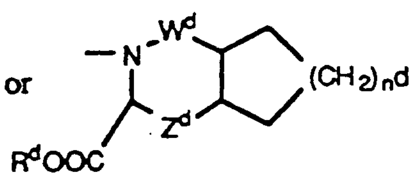

where:where:

$X^d$ and $Y^d$ taken together are $-CH_2-CH_2-$; $-CH(R^{5d})-S-$; $-C(O)-CH_2-$; $-CH_2C(O)-$; $-C(O)-O-$; $C(O)-S-$; $-CH_2-CH(OR^{4d})-$; $-C(O)-N(R^{4d})-$; or $-CH_2-C(R^{4d})-R^{5d}$;

| | |
|---|---|
| $R^{4d}$ is | hydrogen; loweralkyl; aryl; substituted aryl; |
| $R^{5d}$ is | hydrogen; loweralkyl; aryl or substituted aryl; |
| $n^d$ is | 1 to 3; |
| $W^d$ is | absent; $-CH_2-$; or $-C(O)-$; |
| $Z^d$ is | $-(CH_2)m^d$, where $m^d$ is 0 to 2, provided that $m^d$ may not be 0 and $W^d$ may not be absent at the same time; and |
| $R^{6d}$ is | hydrogen; loweralkyl; halo; or $OR^{4d}$; |
| $R^{2d}$ is | $----(CH_2)_r{}^d----B^d----(CH_2)_s{}^d----NR^{7d}R^{15d}$ |

where

$r^d$ and $s^d$ are independently 0 to 3;

$B^d$ is absent; -O-; -S-; or $-NR^{8d}$;

where $R^{8d}$ is hydrogen; loweralkyl; alkanoyl; or aroyl; and

$R^{7d}$ is

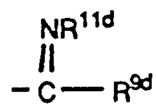 ; 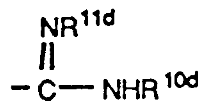 ; or 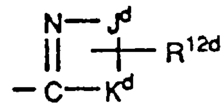

where

$R^{9d}$ is loweralkyl; aralkyl; aryl; heteroaryl; or heteroarloweralkyl and these groups substituted by hydroxy, lower alkoxy or halo; carboxyl; carboxamido; nitromethenyl.

$R^{10d}$ is hydrogen; loweralkyl; aryl; or amidino;

$R^{11d}$ is hydrogen; loweralkyl; cyano; amidino; aryl; aroyl; loweralkanoyl; -C(O)-NHR$^{13d}$;-C(O)-OR$^{13d}$; -$NO_2$; -$SO_2NH_2$; or $SO_2R^{13d}$;

$R^{12d}$ is hydrogen; loweralkyl; halo; aralkyl; amino; cyano; mono- or diloweralkylamino; or OR$^{4d}$;

$R^{13d}$ is hydrogen; loweralkyl; or aryl;

$R^{15d}$ is hydrogen; lower alkyl; aralkyl; or aryl;

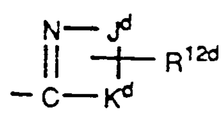

constitute a basic heterocycle of 5 or 6 atoms or benzofused analogs thereof and optionally containing 1-3 N atoms, an oxygen, a sulfur, an S=O, or an $SO_2$ group optionally substituted by amino, lower alkyl amino, diloweralkyl amino, lower alkoxy, or aralkyl groups;

$R^{3d}$ is $C_{3-8}$ cycloalkyl and benzofused $C_{3-8}$ cycloalkyl; perhydrobenzofused $C_{3-8}$ cycloalkyl; aryl; substituted aryl; heterartyl; substituted heteroaryl;

$R^{14d}$ is hydrogen or loweralkyl; and, a pharmaceutically acceptable salt thereof;

European Patent 79022 published May 18, 1983 discloses N-amino acyl-azabicyclooctane carboxylic acid derivatives which have the formula

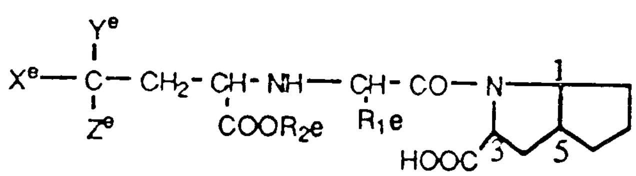

hydrogen atoms at ring positions 1 and 5 are cis to each other and the 3-carboxy group has the endo orientation;

$R_1{}^e$ is H, allyl, vinyl or the side chain of an optionally protected naturally occurring α-amino acid;

$R_2{}^e$ is H, 1-6C alkyl, 2-6C alkenyl or aryl(1-C alkyl);

$Y^e$ is H or OH and $Z^e$ is H, or $Y^e$ and $Z^e$ together oxygen;

$X^e$ is 1-6C alkyl, 2-6C alkenyl, 5-9C cycloalkyl, 6-12C aryl (optionally substituted by one to three 1-4C alkyl or alkoxy, OH, halo, nitro, amino (optionally substituted by one or two 1-4C alkyl), or methylenedioxy) or indol-3-yl);

European Patent 46953 published March 10, 1982 discloses N-amino acyl-indoline and tetrahydro isoquinoline carboxylic acids which are angiotensin coverting enzyme inhibitors and have the formula

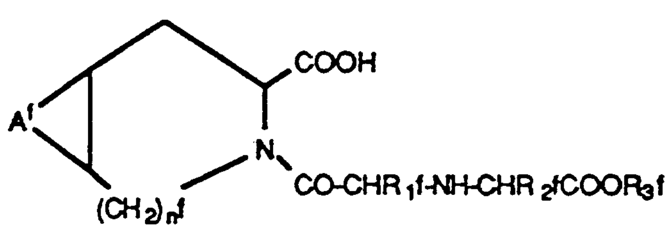

$n^f$ is 0 or 1;

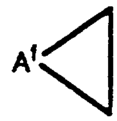

is a benzene or cyclohexane ring:

$R_1{}^f$ and $R_2{}^f$ are each 1-6C alkyl, 2-6C alkenyl, 5-7C cycloalkyl, 5-7C cycloalkenyl, 7-12C cydoalkylalkyl, optionally partially hydrogenated 6-10C aryl, 7-14C aralkyl or 5-7 membered monocyclic or 8-10 membered bicyclic heterocyclyl containing 1 or 2 S or O and/or 1-4N atoms; all $R_1{}^f$ and $R_2{}^f$ groups are optionally substituted,

$R_3{}^f$ is H, 1-6C alkyl, 2-6C alkenyl or 7-14C aralkyl.

The following Table II lists ACE inhibitors preferred for use in the combination of this invention.

## TABLE II
## PREFERRED ACE INHIBITORS

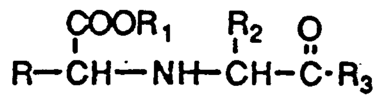

| | R | $R_1$ | $R_2$ | $R_3$ |
|---|---|---|---|---|
| spirapril | $C_6H_5CH_2CH_2$- | Et | $CH_3$ | 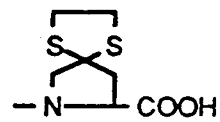 |
| enalapril | $C_6H_5CH_2CH_2$- | Et | $CH_3$ | prolyl |
| ramipril | $C_6H_5CH_2CH_2$- | Et | $CH_3$ | 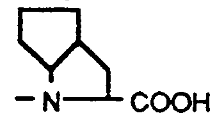 |
| perindopril | $CH_3CH_2CH_2$- | Et | $CH_3$ | 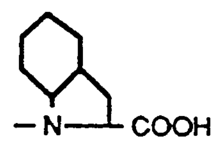 |
| indolapril | $C_6H_5CH_2CH_2$- | Et | $CH_3$ | 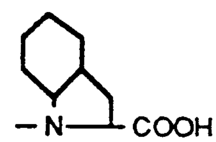 |
| lysinopril | $C_6H_5CH_2CH_2$- | H | $NH_2(CH_2)_4$- | prolyl |
| quinapril | $C_6H_5CH_2CH_2$- | Et | $CH_3$ | 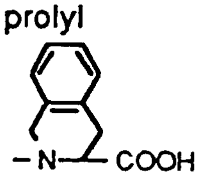 |
| pentopril (NH = $CH_2$) | $CH_3$ | Et | $CH_3$ | 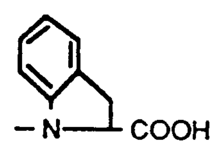 |
| cilazapril | $C_6H_5CH_2CH_2$- | H | 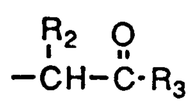 is | 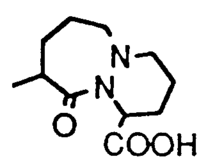 |

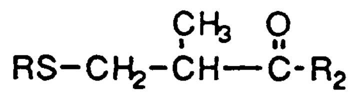

| | R | R2 |
|---|---|---|
| captopril | H | prolyl |
| zofenopril | $C_6H_5CO-$ | 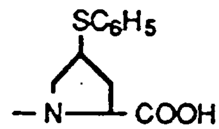 |
| pivalopril | 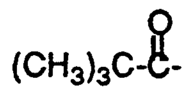 | 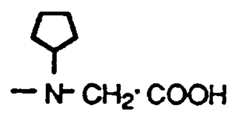 |

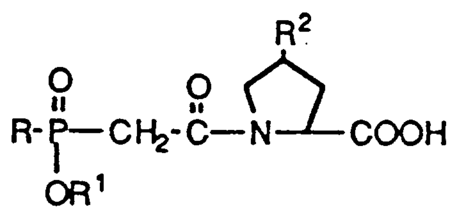

| | R | R1 | R2 |
|---|---|---|---|
| fosinopril | $C_6H_5-(CH_2)_4-$ | 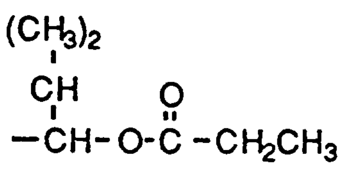 | $C_6H_5-$ |

Compounds of the present invention can be made by methods well known to those skilled in the art. For example, a mercaptan of formula III can be oxidized to the disulfide and converted to an acid of formula IV, then amidated with an amino acid, amino ester or amino amide of formula V to obtain a compound of formula I:

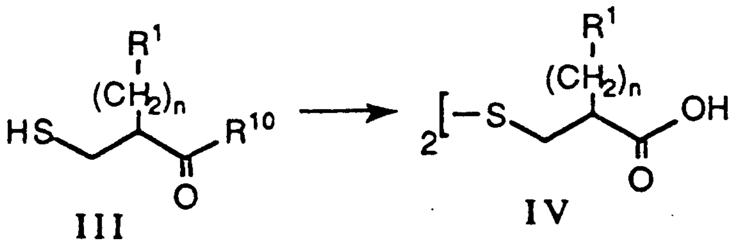

III IV

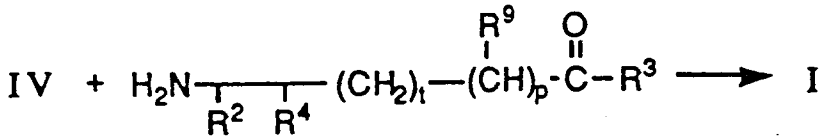

V

wherein n, p, t, $R^1$, $R^2$, $R^3$, $R^4$ and $R^9$ are as defined above and $R^{10}$ is OH or a group convertible to OH, e.g. ethoxy or benzyloxy. An analogous procedure using an acid of formula VI and an amine of formula VII can be used to prepare compounds of formula II:

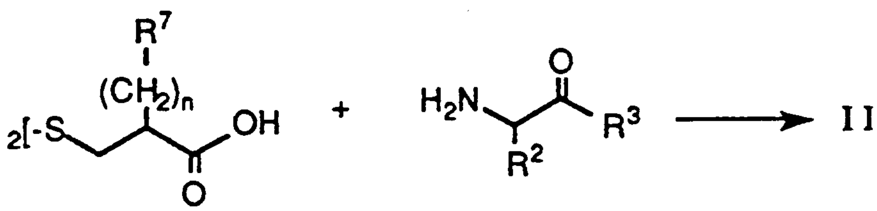

VI VII

wherein n, $R^2$, $R^3$, and $R^7$ are as defined above.

A second method for preparing compounds of the present invention above comprises oxidizing a mercapto-acylamino acid of formula Ia or IIa to obtain the disulfide:

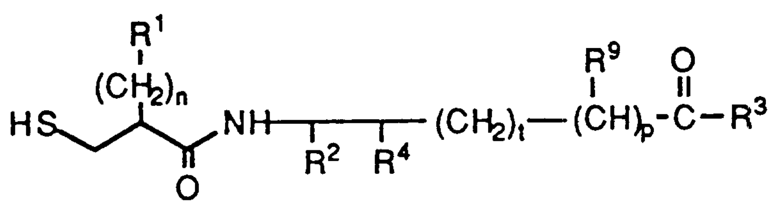

Ia

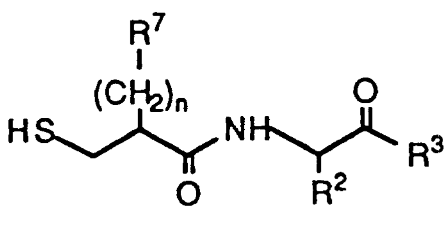

The reaction can be carried out in an organic solvent such as ethanol using an oxidizing agent such as iodine.

Starting materials of formulae Ia and IIa can be made by methods well known to those skilled in the art. A typical general procedure for preparing compounds of formula Ia is to combine a propionic acid, IIIa (i.e., a compound of formula III wherein $R^{10}$ is OH), with an amino acid, amino ester or amino amide, V, under typical peptide coupling conditions, using, for example, a coupling agent such as 1-(3-dimethylaminopropyl)-3-ethylcarbodiimide hydrochloride (EDC):

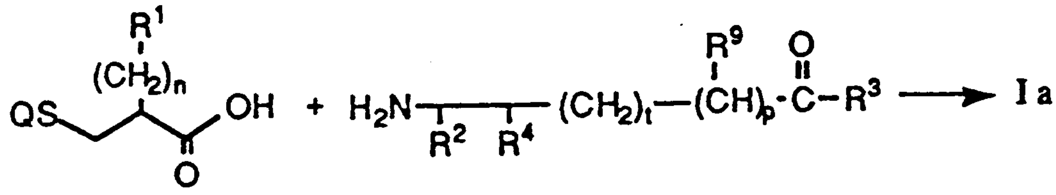

IIIa V Ia

wherein Q is a sulfur-protecting group such as acetyl or benzoyl and $R^3$ is as defined above. To obtain compounds of formula Ia, the sulfur protecting group can be removed by conventional means, e.g. removal of an acetyl or benzoyl group can be accomplished by treating with sodium hydroxide, then acidifying with HCl.

Alternatively, the propionic acid (IIIa) can be converted by known methods (e.g. treatment with thionyl chloride) to the corresponding acid chloride (IIIb), and the acid chloride can be reacted with the amino acid, amino ester or amino amide in the presence of a base such as triethylamine to obtain, after deprotection, a compound of formula Ia:

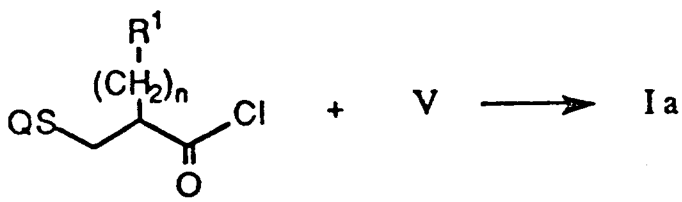

IIIb V Ia

For compounds of formula V wherein $R^4$ is hydroxy, it may be desirable to protect such a group during the reaction, e.g. with a t-butoxycarbonyl or benzyloxycarbonyl group.

Similar reactions may be used to prepare compounds of formula IIa, using a propionic acid of formula VIa (or the corresponding acid chloride) wherein Q is as defined above, and an amino acid, amino ester or amide of formula VII:

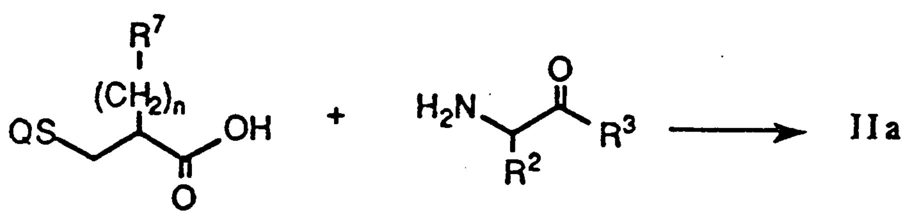

VIa VII IIa

It is apparent that using methods well known to those skilled in the art, compounds of formula Ia and IIa can be converted to different compounds of formula Ia and IIa, respectively, by appropriate reaction of the $R^3$ variable, e.g. an acid can be converted to an amide or an ester to an amide.

Compounds of formula III, IIIa, and V to VII are known in the art or can be prepared by methods well known in the art.

We have found that the novel compounds of the present invention are effective in treating cardiovascular disorders such as congestive heart failure, edema, renal insufficiency and various types of hypertension, particularly volume expanded hypertension. These novel compounds enhance both the magnitude and duration of the antihypertensive and natriuretic effects of endogenous ANF. Administration of a combination of a mercaptoacylamino acid disulfide derivative and an ACE inhibitor provides an antihypertensive and anticongestive heart failure erect greater than either the mercapto-acylamino acid disulfide derivative or ACE inhibitor alone. Administration of a combination of a mercaptoacylamino acid disulfide derivative of

formula I or II and an exogenous ANF or ACE inhibitor is therefore particularly useful in treating hypertension or congestive heart failure.

Thus, the compounds of the present invention are useful for treating cardiovascular disorders, viz a mercaptoacylamino acid disulfide derivative of formula I or II or a mercaptoacylamino acid disulfide derivative of formula I or II in combination with an ANF or an ACE inhibitor, can be administered to a mammal in need of such treatment. The drug or combination of drugs is preferably administered in a pharmaceutically acceptable carrier, e.g. for oral or parenteral administration. The combinations of drugs may be co-adminstered in a single composition, or components of the combination therapy may be administered separately. Where the components are administered separately, any convenient combination of dosage forms may be used, e.g. oral mercaptoacylamino acid disulfide derivative/oral ANF, oral mercaptoacylamino acid disulfide derivative/parenteral ACE inhibitor, parenteral mercaptoacylamino acid disulfide derivative/oral ANF, parenteral mercaptoacylamino acid disulfide derivative/parenteral ACE inhibitor.

When the components of a combination of a mercapto-acylamino acid disulfide derivative and an ANF are administered separately, it is preferred that the mercaptoacylamino acid disulfide derivative be administered first.

The present invention also relates to a pharmaceutical composition comprising a mercaptoacylamino acid disulfide derivative for use in treating hypertension or congestive heart failure, to a pharmaceutical composition comprising both a mercaptoacylamino acid disulfide derivative and an ANF and to a pharmaceutical composition comprising both a mercaptoacylamino acid disulfide derivative and an ACE inhibitor.

The antihypertensive effect of mercaptoacylamino acid disulfide derivatives was determined according to the following procedure:

Male Sprague Dawley rats weighing 100-150 g were anesthetized with ether and the right kidney was removed. Three pellets containing DOC acetate (desoxycorticosterone acetate, DOCA, 25 mg/pellet) were implanted subcutaneously. Animals recovered from surgery, were maintained on normal rat chow and were allowed free access to a fluid of 1% NaCl and 0.2% KCl instead of tap water for a period of 17-30 days. This procedure results in a sustained elevation in blood pressure and is a slight modification of published procedures (e.g. Brock et al., 1982) that have been used to produce DOCA salt hypertension in the rat.

On the day of study, animals were again anesthetized with ether and the caudal artery was cannulated for blood pressure measurement. Patency of the caudal artery cannula was maintained with a continuous infusion of dextrose in water at a rate of 0.2 ml/hr. Animals were placed into restraining cages where they recovered consciousness. Blood pressure was measured from caudal artery catheter using a Statham pressure transducer attached to a Beckman oscillographic recorder. In addition, a cardiovascular monitoring device (Buxco Electronics, Inc.) and a digital computer were used to calculate average blood pressures.

After an equilibration period of at least 1.5 hr., animals were dosed subcutaneously (1 ml/kg) with vehicle (methylcellulose, hereinafter MC) or mercaptoacylamino acid disulfide derivative and blood pressure was monitored for the next 4 hours.

A similar procedure can be used to determine the effect of mercaptoacylamino acid disulfide derivatives in combination with ACE inhibitors.

The antihypertensive effect of mercaptoacylamino acid disulfide derivatives in combination with ANF can be determined according to the following procedures:

Male spontaneously hypertensive rats (SHR), 16-18 weeks old, 270-350 g, are anesthetized with ether and the abdominal aorta is cannulated through the tail artery. The animals are then placed into restrainers to recover from anesthesia (in less than 10 min.) and remain inside throughout the experiments. Through a pressure transducer (Gould P23 series) analog blood pressure signals are registered on a Beckman 612 recorder. A Buxco digital computer is used to obtain mean arterial pressures. Patency of the arterial cannula is maintained with a continuous infusion of 5% dextrose at 0.2 ml/hr. Animals are allowed a 90-min equilibration period. The animals first undergo a challenge with an ANF such as atriopeptin II (AP II) or AP28 30 µg/kg iv and at the end of 60 min. are treated with drug vehicle or a mercapto-acylamino acid subcutaneously. A second ANF challenge is administered 15 min. later and blood pressure is monitored for the next 90 min.

The antihypertensive effect in SHR of mercaptoacylamino acid disulfide derivatives and ACE inhibitors, alone and in combination, can be determined as follows:

Animals are prepared for blood pressure measurement as described above. After stabilization, animals are dosed subcutaneously or orally with test drugs or placebo and blood pressure is monitored for the next 4 hr.

The compounds having structural formulae I and II have also been found to inhibit the activity of enzymes designated enkephalinases. The compounds are particularly useful for the inhibition of enkephalinase A, which is derived from the striata of both rats and humans. In in vitro tests, using test procedures for enkephalinase A inhibition well known to those skilled in the art, selected compounds having structural formula I and II have been found to inhibit the activity of the aforementioned enzyme. Therefore, the present invention also relates to a method of inhibiting the action of enkephalinases in a mammal thereby to elicit an analgesic effect with a compound of formula I or II, and to analgesic pharmaceutical compositions comprising compounds of formula I or II.

The use of atrial natriuretic peptides in the treatment of nephrotoxicity associated with the immunosuppressive cyclosporin was reported by Capasso et al in the American Journal of Hypertension, 3, 3 (1990), p. 204-210. Since compounds of this invention enhance endogenous ANF, they can be used alone to treat nephrotoxicity, or they can be administered in combination with exogenous ANF.

The compositions of this invention comprise a mercapto-acylamino acid disulfide derivative or a mercaptoacylamino acid disulfide derivative and an ANF or a mercaptoacylamino acid disulfide derivative and an ACE inhibitor in combination with a pharmaceutically acceptable carrier for administration to mammals. A variety of pharmaceutical forms is suitable, preferably for oral or parenteral administration, although mechanical delivery systems such as transdermal dosage forms are also contemplated.

The daily dose of the compound or combinations of this invention for treatment of hypertension or congestive heart failure is as follows: for mercaptoacylamino acid disulfide derivatives alone the typical dosage is 0.1 to 10 mg/kg of mammalian weight per day administered in single or divided dosages; for the combination of mercaptoacylamino acid disulfide derivative and an ANF, the typical dosage is 0.1 to 10 mg of mercaptoacylamino acid disulfide derivative/kg mammalian weight per day in single or divided dosages plus 0.001 to 0.1 mg ANF/kg of mammalian weight per day, in single or divided dosages, and for the combination of mercaptoacylamino acid disulfide derivative and an ACE inhibitor, the typical dosage is 0.1 to 10 mg of mercaptoacylamino acid disulfide derivative/kg mammalian weight per day in single or divided dosages plus 0.1 to 30 mg ACE inhibitor/kg of mammalian weight per day in single or divided dosages. The exact dose of any component or combination to be administered is determined by the attending clinician and is dependent on the potency of the compound administered, the age, weight, condition and response of the patient.

Generally, in treating humans having hypertension or congestive heart failure, the compounds or combinations of this invention may be administered to patients in a dosage range as follows: for treatment with mercaptoacylamino acid disulfide derivatives alone, 5 to 500 mg per dose given 1 to 4 times a day, giving a total daily dose of 5 to 2000 mg per day; for the combination of mercaptoacylamino acid disulfide derivative and ANF, 5 to 500 mg mercaptoacylamino acid disulfide derivative per dose given 1 to 4 times a day and 0.001 to 1 mg ANF given 1 to 6 times a day (total daily dosage range of 5 to 2000 mg day and .001 to 6 mg/day, respectively); and for the combination of a mercaptoacylamino acid disulfide derivative and an ACE inhibitor, 5 to 500 mg mercaptoacylamino acid disulfide derivative per dose given 1 to 4 times a day and 5 to 50 mg ACE inhibitor given 1 to 3 times a day (total daily dosage range of 5to 2000 mg/day and 5 to 150 mg/day, respectively). Where the components of a combination are administered separately, the number of doses of each component given per day may not necessarily be the same, e.g. where one component may have a greater duration of activity, and will therefore need to be administered less frequently.

To produce an analgesic effect, compounds of this invention will be administered in a dosage range of from 1 to 100 mg/kg. The doses are to be administered at intervals of from 3 to 8 hours. However, the quantity and frequency of dosage will depend upon such factors as the severity of the pain, the general physical condition of the patient, the age and weight of the patient, and other factors recognized by the skilled clinician.

For treatment of edema, renal insufficiency or nephrotoxicity associated with immunosuppressive therapy, dosage ranges of the compounds of this invention are the same as for treatment of hypertension with the use of mercapto-acylamino acid disulfide derivatives of this invention alone or in combination with ANF.

Typical oral formulations include tablets, capsules, syrups, elixirs and suspensions. Typical injectable formulations include solutions and suspensions.

The typical acceptable pharmaceutical carriers for use in the formulations described above are exemplified by: sugars such as lactose, sucrose, mannitol and sorbitol, starches such as cornstarch, tapioca starch and potato starch; cellulose and derivatives such as sodium carboxymethyl cellulose, ethyl cellulose and methyl cellulose; calcium phosphates such as dicalcium phosphate and tricalcium phosphate; sodium sulfate; calcium sulfate; polyvinylpyrrolidone, polyvinyl alcohol; stearic acid; alkaline earth metal stearates

such as magnesium stearate and calcium stearate, stearic acid, vegetable oils such as peanut oil, cottonseed oil, sesame oil, olive oil and corn oil; non-ionic, cationic and anionic surfactants; ethylene gylcol polymers; beta-cyclodextrin; fatty alcohols and hydrolyzed cereal solids; as well as other nontoxic compatible fillers, binders, disintegrants, buffers, preservatives, antioxidants, lubricants and flavoring agents commonly used in pharmaceutical formulations.

Since the present invention relates to treatment of hypertension or congestive heart failure with a combination of active ingredients wherein said active ingredients may be administered separately, the invention also relates to combining separate pharmaceutical compositions in kit form. That is, two kits are contemplated, each combining two separate units: a mercapto-acylamino acid disulfide derivative pharmaceutical composition and an ANF pharmaceutical composition in one kit and a mercapto-acylamino acid disulfide derivative pharmaceutical composition and an ACE inhibitor pharmaceutical composition in a second kit. The kit form is particularly advantageous when the separate components must be administered in different dosage forms (e.g. oral and parenteral) or are administered at different dosage intervals.

Following are examples of procedures for preparing compounds of formulae 1 and 2.

PREPARATION 1

N-[3-MERCAPTO-2(S)-(2-METHYLBENZYL)PROPIONYL]-(S)-METHIONINE ETHYL ESTER

To N-[3-acetylthio-2(S)-(2-methylbenzyl)propionyl]-(S)-methionine ethyl ester (1.00 g), add 6.8% ammonia in absolute ethanol (20 ml) and stir for 3 hr. Concentrate the resulting solution and dry the resulting residue in vacuo to obtain a white solid, m.p. 73-76°C, $[\alpha]_D^{26}$ = -7.6° (MeOH).

PREPARATION 2

N-[3-MERCAPTOMETHYL-2(S)-(2-METHYLBENZYL) PROPIONYL]-(S)-ISOSERINE ETHYL ESTER

Under a nitrogen atmosphere, add 7.8% w/w $NH_3$ in EtOH to N-[2(S)-acetylthiomethyl-3-(2-methylphenyl)propionyl]-(S)-isoserine. ethyl ester (2.62 g) and stir for 15 hr. Concentrate the reaction mixture in vacuo to obtain a colorless oil. Treat the oil with degassed water and concentrate in vacuo to obtain the title compound as a colorless oil, $[\alpha]_D{}^{26}$ = +60.7°C (MeOH).

EXAMPLE 1

1,1'-[DITHIOBIS-[2(S)-(2-METHYLBENZYL)-1-OXO-3,1-PROPANEDIYL]]BIS-(S)-METHIONINE DIETHYL ESTER

To the product of Preparation 1 (0.89 g) in absolute EtOH (30 ml), add 1% iodine solution in absolute EtOH (~32 ml) dropwise (until light brown solution is obtained). Evaporate the ethanol and partition the residue between diethyl ether/1% sodium thiosulfate solution. Partition the organic solution with brine. Dry ($MgSO_4$) and concentrate to obtain a white solid, m.p. 74-79°C, $[\alpha]_D^{26}$ = -176.4° (MeOH).

EXAMPLE 2

1,1'-[DITHIOBIS-[2(S)-(2-METHYLBENZYL)-1-OXO-3,1-PROPANEDIYL]]BIS-(S)-METHIONINE

Add 1N NaOH (1.3 ml) to the product of Example 1 (0.46 g) and stir the resulting mixture for 3 hr. Add 0.1N HCl and extract with EtOAc. Concentrate the dried ($MgSO_4$) EtOAc and chromatograph the residue on preparative thin layer plates (4 x 1000μ) using $CH_2Cl_2$:$NH_4OH$:MeOH 70:27:3 as eluant to obtain a light yellow solid, m.p. 53-58°C, $[\alpha]_D^{26}$ = -177.8° (MeOH).

EXAMPLE 3

1,1'-[DITHIOBIS-[2(S)-(2-METHYLBENZYL)-1-OXO-3,1-PROPANEDIYL]]BIS-(S)-ISOSERINE DIETHYL ESTER

Dissolve the product of Preparation 2 (1.72 g) in a 1% iodine solution in EtOH (70ml) and stir the reaction mixture at room temperature for 18 hr. Concentrate the mixture in vacuo and add EtOAc (800 ml).

Partition the EtOAc solution with 5% sodium bicarbonate solution, 1% sodium thiosulfate solution and then brine. Concentrate the dried ($MgSO_4$) EtOAc solution in vacuo to give a white solid. Place the white solid on a column of flash silica gel (200 ml) and elute with EtOAc:hexane 1:1 (2000ml), 3:2 (2000 ml), 3:1 (2000 ml), EtOAc (1000 ml) and EtOAc:MeOH 4:1 (2000 ml) to give a white solid. Place this white solid on a column of flash silica gel (120 g) and elute with $CH_2Cl_2$:MeOH 99:1 (2000 ml), 49:1 (2000 ml) and 19:1 (2000 ml) to obtain the title compound as a white solid, mp 137-8 ° C, $[\alpha]^{26}_D = -1.5°$ (MeOH).

EXAMPLE 4

1,1'-[DITHIOBIS-[2(S)-(2-METHYLBENZYL)-1-OXO-3,1-PROPANEDIYL]]BIS-(S)-ISOSERINE

Add 1N NaOH (1.1 ml) to the product of Example 3 (0.324 g) in MeOH (15 ml) and stir for 2 hr. Concentrate the reaction mixture under a stream of nitrogen and add 1N HCl (2 ml) to give a white precipitate. Filter and wash the solid with water and dry in vacuo to obtain the title compound as a white solid, m.p. 121-3 ° C, $[\alpha]_D^{26} = -30.3°$ (MeOH).

Using the test procedures described above, compounds of examples 1 to 4 were found to produce a drop in blood pressure (ΔBP) in the DOCA salt model and in the ANF potentiation procedure:

| | ANF Potentiation | | | DOCA Salt | | |
|---|---|---|---|---|---|---|
| Compound | ΔBP (mmHg) | mg/kg | route | ΔBP (mmHg) | mg/kg | route |
| Example 1 | 0 | 30 | sc | 15 | 3 | po |
| Example 2 | 27 | 30 | sc | 57 | 10 | po |
| | | | | 41 | 1 | po |
| Example 3 | 29 | 30 | sc | 44 | 10 | po |
| | | | | 30 | 1 | po |
| | | | | 31 | 0.1 | po |
| Example 4 | 24 | 30 | sc | 31 | 10 | po |
| | | | | 21 | 1 | po |
| | | | | 0 | 0.1 | po |
| (sc = subcutaneous; po = oral) | | | | | | |

The following formulations exemplify some of the dosage forms of the compositions of this invention. In each, the term "active ingredient" designates a compound of formula I or II, preferably 1,1'-[dithiobis-[2(S)-(2-methylbenzyl)-1-oxo-3,1-propanediyl]]bis-(S)-methionine. However, this compound can be replaced by equally effective amounts of other compounds of formula I or II.

Example A

| Tablets | | | |
|---|---|---|---|
| No. | Ingredient | mg/tablet | mg/tablet |
| 1 | Active Compound | 100 | 500 |
| 2 | Lactose USP | 122 | 113 |
| 3 | Corn Starch, Food Grade, as a 10% Paste in Purified Water | 30 | 40 |
| 4 | Corn Starch, Food Grade | 45 | 40 |
| 5 | Magnesium Stearate | 3 | 7 |
| | Total | 300 | 700 |

Method of Manufacture

Mix items Nos. 1 and 2 in suitable mixer for 10-15 minutes. Granulate the mixture with item No. 3. Mill the damp granules through a coarse screen (e.g., 1/4", 0.63 cm) if necessary. Dry the damp granules. Screen the dried granules if necessary and mix with Item No. 4 and mix for 10-15 minutes. Add Item No. 5 and mix for 1-3 minutes. Compress the mixture to appropriate size and weigh on a suitable tablet machine.

Example B

| Capsules | | | |
|---|---|---|---|
| No. | Ingredient | mg/capsule | mg/capsule |
| 1 | Active Compound | 100 | 500 |
| 2 | Lactose USP | 106 | 123 |
| 3 | Corn Starch, Food Grade | 40 | 70 |
| 4 | Magnesium Stearate NF | 4 | 7 |
| | Total | 250 | 700 |

Method of Manufacture

Mix Item Nos. 1, 2 and 3 in a suitable blender for 10-15 minutes. Add Item No. 4 and mix for 1-3 minutes. Fill the mixture into suitable two-piece hard gelatin capsules on a suitable encapsulating machine.

Example C

| Parenteral Preparation | | |
|---|---|---|
| Ingredient | mg/vial | mg/vial |
| Active Compound Sterile Powder | 100 | 500 |

For reconstitution add sterile water for injection or bacteriostatic water for injection.

## Claims

**Claims for the following Contracting States : AT, BE, CH, DE, DK, FR, GB, IT, LI, LU, NL, SE**

1. A compound having the structural formula

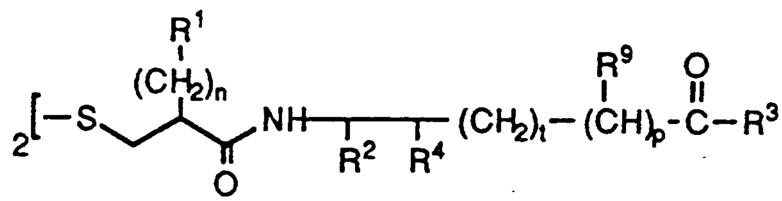

I

or

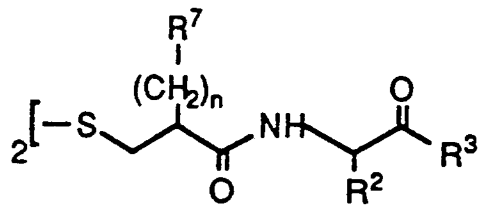

II

wherein

$R^1$ is C$_1$-C$_6$ alkyl, C$_3$-C$_6$ cyclo alkyl, aryl or heteroaryl;

$R^2$ is hydrogen; C$_1$-C$_6$ alkyl; C$_3$-C$_6$ cyclo alkyl; C$_1$-C$_6$ alkyl substituted with hydroxy, C$_1$-C$_6$ alkoxy, mercapto, C$_1$-C$_6$ alkylthio, aryl, heteroaryl, aralkyloxy or aralkylthio; aryl; or heteroaryl;

$R^3$ is $-OR^5$ or $-NR^5R^6$;

$R^4$ and $R^9$ are independently $-(CH_2)_qR^8$;

$R^5$ and $R^6$ are independently selected from the group consisting of hydrogen, C$_1$-C$_6$ alkyl, hydroxy C$_1$-C$_6$ alkyl, C$_1$-C$_6$ alkoxy C$_1$-C$_6$ alkyl and aryl C$_1$-C$_6$ alkyl, or $R^5$ and $R^6$ together with the nitrogen to which they are attached form a 5-7 membered ring;

$R^7$ is phenyl substituted by 1 to 3 substituents selected from the group consisting of C$_1$-C$_6$ alkyl, C$_1$-C$_6$ alkoxy, cycloalkyl, cyano and aminomethyl;

$R^8$ is hydrogen, hydroxy, C$_1$-C$_6$ alkoxy, mercapto, C$_1$-C$_6$ alkylthio, aryl or heteroaryl;

wherein aryl is a mono-cyclic or fused ring bicyclic carbocyclic aromatic group having 6 to 10 ring members and heteroaryl is a mono-cyclic or fused ring bicyclic aromatic group having 5 to 10 ring members wherein 1-2 ring members are independently nitrogen, oxygen or sulfur, and wherein the carbon ring members of the aryl and heteroaryl groups are substituted by 0-3 substituents selected from C$_1$- C$_6$ alkyl, hydroxy, halo, C$_1$- C$_6$ alkoxy, C$_3$- C$_6$ cyclo alkyl, cyano, aminomethyl, trifluoromethyl, phenyl, phenoxy or phenylthio;

n is 1 or 2;

p is 0 or 1;

q is 0, 1 or 2; and

t is 0 or 1;

or a pharmaceutically acceptable salt thereof.

2. A compound of formula I of claim 1 wherein $R^1$ is phenyl or C$_1$-C$_6$ alkyl substituted phenyl and n is 1.

3. A compound of formula I of claim 1 wherein $R^3$ is hydroxy or C$_1$-C$_6$ alkoxy.

4. A compound of formula I of claim 1 wherein p is zero.

5. A compound of formula I of claim 1 wherein $R^4$ is hydrogen, hydroxy, methoxy, phenyl or benzyl.

6. A compound of formula I of claim 1 wherein $R^2$ is hydrogen or thienyl.

7. A compound of formula I of claim 1 wherein p and t are each zero, $R^2$ is hydrogen and $R^4$ is hydroxy or methoxy.

8. A compound of formula II of claim 1 wherein $R^7$ is $C_1$-$C_6$ alkyl-substituted phenyl and n is 1.

9. A compound of formula II of claim 1 wherein $R^2$ is $C_1$-$C_6$ alkylthio $C_1$-$C_6$ alkyl.

10. A compound of claim 1 which is:
1,1'-[dithiobis-[2(S)-(2-methylbenzyl)-1-oxo-3,1-propanediyl]]-bis-(S)-isoserine diethyl ester;
1,1'-[dithiobis-[2(S)-(2-methylbenzyl)-1-oxo-3,1-propanediyl]]-bis-(S)-isoserine;
1,1'-[dithiobis[2(S)-(2-methylbenzyl)-1-oxo-3,1-propanediyl]]bis-(S)-methionine diethyl ester;
1,1'-[dithiobis[2(S)-(2-methylbenzyl)-1-oxo-3,1-propanediyl]]bis-(S)-methionine.

11. A pharmaceutical composition for treating hypertension, congestive heart failure, edema, renal insufficiency, nephrotoxicity or pain comprising an effective amount of a compound of claim 1, alone or in combination with an atrial nartiuretic factor or an angiotensin converting enzyme inhibitor, in a pharmaceutically acceptable carrier.

12. A composition of claim 11 wherein the atrial natriuretic peptide is chosen from α human AP 21, α human AP 28, α human AP 23, α human AP 24, α human AP 25, α human AP 26, α human AP 33, and the corresponding atrial peptides wherein the methionine at position 12 is replaced by isoleucine.

13. A composition of claim 11 wherein the angiotensin converting enzyme inhibitor is selected from: spirapril, enalapril, ramipril, perindopril, indolapril, lysinopril, quinapril, pentopril, cilazapril, captopril, zofenopril, pivalopril and fosinopril.

14. A kit comprising in separate containers in a single package pharmaceutical compositions for use in combination to treat hypertension, congestive heart failure or nephrotoxicity in mammals which comprises in one container a pharmaceutical composition comprising a mercapto-acylamino acid disulfide derivative of claim 1 and in a second container a pharmaceutical composition comprising an atrial natriuretic factor.

15. A kit comprising in separate containers in a single package pharmaceutical compositions for use in combination to treat hypertension or congestive heart failure in mammals which comprises in one container a pharmaceutical composition comprising a mercapto-acylamino acid disulfide derivative of claim 1 and in a second container a pharmaceutical composition comprising an angiotensin converting enzyme inhibitor.

16. A method for preparing a pharmaceutical composition comprising admixing a compound of claim 1 with a pharmaceutically acceptable carrier.

17. The use of a compound of claim 1 for the manufacture of a medicament for treating hypertension, congestive heart failure, edema, renal insufficiency, nephrotoxicity or pain.

18. A process for the preparation of a compound of formula I or II as defined in claim 1, wherein $R^1$, $R^2$, $R^3$, $R^4$, $R^7$, $R^9$, n, p and t are as defined in claim 1, selected from the following processes A, B, C, and D:
Process A for preparing compounds of formula I comprising amidating an acid of formula IV with an amine of formula V:

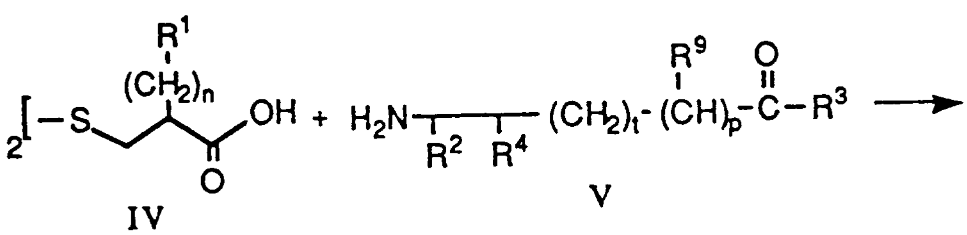

IV V I

Process B for preparing compounds of formula II comprising amidating an acid of formula VI with an amine of formula VII:

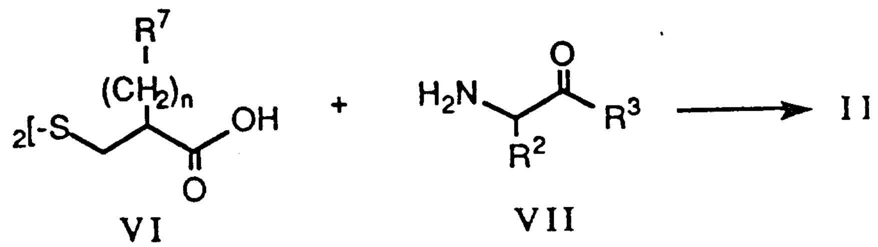

VI VII

Process C for preparing compounds of formula I comprising oxidizing a mercaptoacylamino acid of formula Ia:

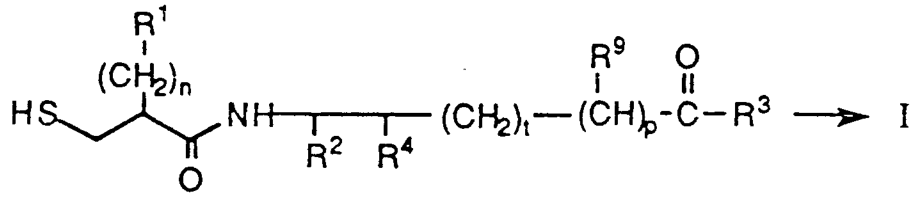

Ia

Process D for preparing compounds of formula II comprising oxidizing a mercaptoacylamino acid of formula IIa:

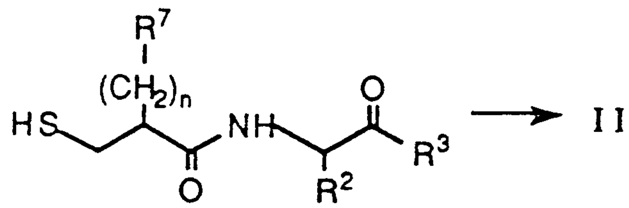

IIa

wherein each process is followed by isolation of the preferred isomer, if desired, and removal of the protecting groups, if necessary, to yield the desired product, and if desired, preparation of a salt thereof.

**Claims for the following Contracting State : ES**

**1.** A process for the preparation of a compound having the structural formula

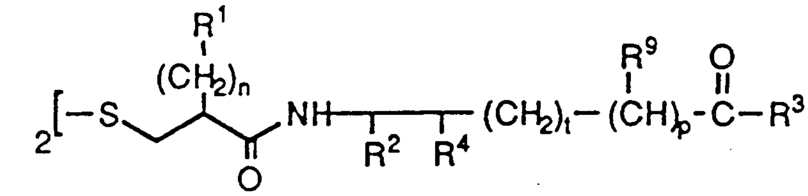

I

or

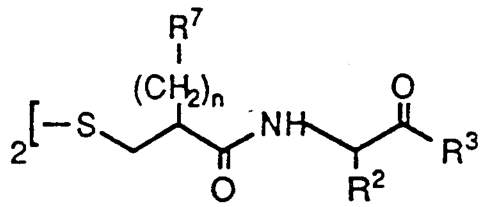

II

wherein

| | |
|---|---|
| $R^1$ is | $C_1$-$C_6$ alkyl, $C_3$-$C_6$ cyclo alkyl, aryl or heteroaryl; |
| $R^2$ is | hydrogen; $C_1$-$C_6$ alkyl; $C_3$-$C_6$ cyclo alkyl; $C_1$-$C_6$ alkyl substituted with hydroxy, $C_1$-$C_6$ alkoxy, mercapto, $C_1$-$C_6$ alkylthio, aryl, heteroaryl, aralkyloxy or aralkylthio; aryl; or heteroaryl; |
| $R^3$ is | $-OR^5$ or $-NR^5R^6$; |
| $R^4$ and $R^9$ are | independently $-(CH_2)_qR^8$; |
| $R^5$ and $R^6$ are | independently selected from the group consisting of hydrogen, $C_1$-$C_6$ alkyl, hydroxy $C_1$-$C_6$ alkyl, $C_1$-$C_6$ alkoxy $C_1$-$C_6$ alkyl and aryl $C_1$-$C_6$ alkyl, or $R^5$ and $R^6$ together with the nitrogen to which they are attached form a 5-7 membered ring; |
| $R^7$ is | phenyl substituted by 1 to 3 substituents selected from the group consisting of $C_1$-$C_6$ alkyl, $C_1$-$C_6$ alkoxy, cycloalkyl, cyano and aminomethyl; |
| $R^8$ is | hydrogen, hydroxy, $C_1$-$C_6$ alkoxy, mercapto, $C_1$-$C_6$ alkylthio, aryl or heteroaryl; |

wherein aryl is a mono-cyclic or fused ring bicyclic carbocyclic aromatic group having 6 to 10 ring members and heteroaryl is a mono-cyclic or fused ring bicyclic aromatic group having 5 to 10 ring members wherein 1-2 ring members are independently nitrogen, oxygen or sulfur, and wherein the carbon ring members of the aryl and heteroaryl groups are substituted by 0-3 substituents selected from $C_1$- $C_6$ alkyl, hydroxy, halo, $C_1$- $C_6$ alkoxy, $C_3$- $C_6$ cyclo alkyl, cyano, aminomethyl, trifluoromethyl, phenyl, phenoxy or phenylthio;

n is 1 or 2;
p is 0 or 1;
q is 0, 1 or 2; and
t is 0 or 1;

or a pharmaceutically acceptable salt thereof, which process being selected from the following processes A, B, C, and D:

Process A for preparing compounds of formula I comprising amidating an acid of formula IV with an amine of formula V:

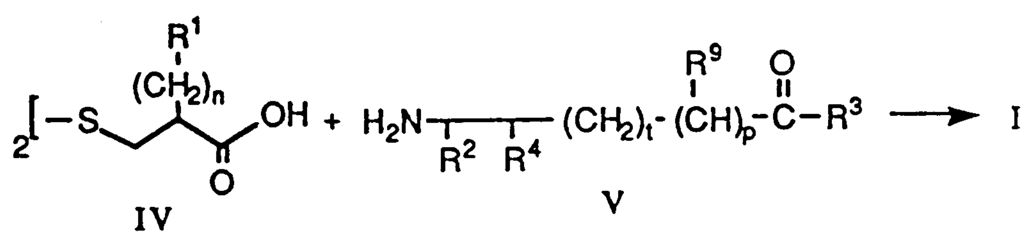

IV V

Process B for preparing compounds of formula II comprising amidating an acid of formula VI with an amine of formula VII:

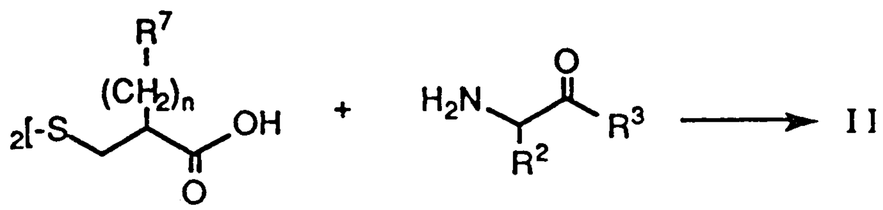

VI VII

Process C for preparing compounds of formula I comprising oxidzing a mercaptoacylamino acid of formula Ia:

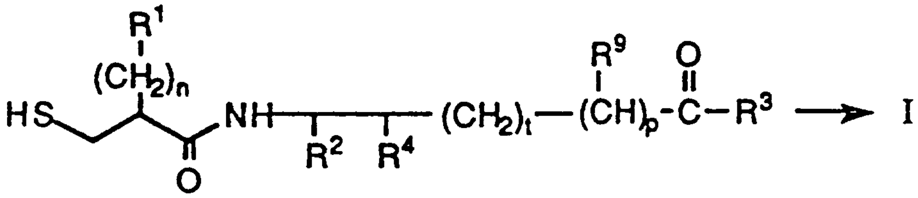

Ia

Process D for preparing compounds of formula II comprising oxidizing a mercaptoacylamino acid of formula IIa:

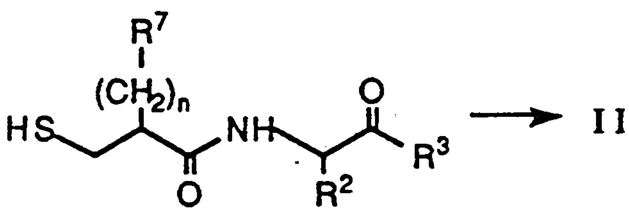

IIa

wherein each process is followed by isolation of the preferred isomer, if desired, and removal of the protecting groups, if necessary, to yield the desired product, and if desired, preparation of a salt thereof.

2. A process of claim 1 wherein in a compound of formula I $R^1$ is phenyl or $C_1$-$C_6$ alkyl substituted phenyl and n is 1.

3. A process of claim 1 wherein in a compound of formula I $R^3$ is hydroxy or $C_1$-$C_6$ alkoxy.

4. A process of claim 1 wherein in a compound of formula I p is zero.

5. A process of claim 1 wherein in a compound of formula I $R^4$ is hydrogen, hydroxy, methoxy, phenyl or benzyl.

6. A process of claim 1 wherein in a compound of formula I $R^2$ is hydrogen or thienyl.

7. A process of claim 1 wherein in a compound of formula I of claim 1 wherein p and t are each zero, $R^2$ is hydrogen and $R^4$ is hydroxy or methoxy.

**8.** A process of claim 1 wherein in a compound of formula II $R^7$ is $C_1$-$C_6$ alkyl-substituted phenyl and n is 1.

**9.** A process of claim 1 wherein in a compound of formula II $R^2$ is $C_1$-$C_6$ alkylthio $C_1$-$C_6$ alkyl.

**10.** A process of claim 1 wherein the produced compound is
1,1'-[dithiobis-[2(S)-(2-methylbenzyl)-1-oxo-3,1-propanediyl]]-bis-(S)-isoserine diethyl ester;
1,1'-[dithiobis-[2(S)-(2-methylbenzyl)-1-oxo-3,1-propanediyl]]-bis-(S)-isoserine;
1,1'-[dithiobis[2(S)-(2-methylbenzyl)-1-oxo-3,1-propanediyl]]bis-(S)-methionine diethyl ester;
1,1'-[dithiobis[2(S)-(2-methylbenzyl)-1-oxo-3,1-propanediyl]]bis-(S)-methionine.

**11.** A method for preparing a pharmaceutical composition for treating hypertension, congestive heart failure, edema, renal insufficiency, nephrotoxicity or pain comprising an effective amount of a compound prepared in accordance with claim 1, alone or in combination with an atrial nartiuretic factor or an angiotensin converting enzyme inhibitor, in a pharmaceutically acceptable carrier, which method comprises admixing the compound preparing in accordance with claim 1 with the pharmaceutically acceptable carrier.

**12.** A method of claim 11 wherein the atrial natriuretic peptide is chosen from $\alpha$ human AP 21, $\alpha$ human AP 28, $\alpha$ human AP 23, $\alpha$ human AP 24, $\alpha$ human AP 25, $\alpha$ human AP 26, $\alpha$ human AP 33, and the corresponding atrial peptides wherein the methionine at position 12 is replaced by isoleucine.

**13.** A method of claim 11 wherein the angiotensin converting enzyme inhibitor is selected from: spirapril, enalapril, ramipril, perindopril, indolapril, lysinopril, quinapril, pentopril, cilazapril, captopril, zofenopril, pivalopril and fosinopril.

**14.** A method for preparing a kit comprising in separate containers in a single package pharmaceutical compositions for use in combination to treat hypertension, congestive heart failure or nephrotoxicity in mammals which method comprises using in one container a pharmaceutical composition comprising a mercapto-acylamino acid disulfide derivative prepared in accordance with claim 1 and in a second container a pharmaceutical composition comprising an atrial natriuretic factor.

**15.** A method for preparing a kit comprising in separate containers in a single package pharmaceutical compositions for use in combination to treat hypertension or congestive heart failure in mammals which method comprises using in one container a pharmaceutical composition comprising a mercapto-acylamino acid disulfide derivativer prepared in accordance with claim 1 and in a second container a pharmaceutical composition comprising an angiotensin converting enzyme inhibitor.

**16.** A method of use of a compound prepared in accordance with claim 1 for the manufacture of a medicament for treating hypertension, congestive heart failure, edema, renal insufficiency, nephrotoxicity or pain.

**Claims for the following Contracting State : GR**

**1.** A compound having the structural formula

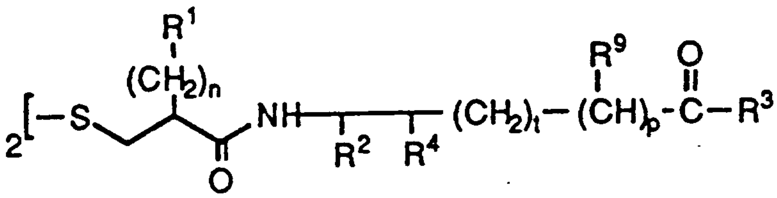

I

or

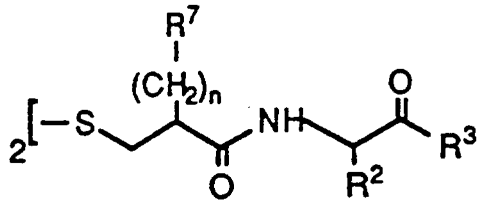

II

wherein

| | |
|---|---|
| $R^1$ is | $C_1$-$C_6$ alkyl, $C_3$-$C_6$ cyclo alkyl, aryl or heteroaryl; |
| $R^2$ is | hydrogen; $C_1$-$C_6$ alkyl; $C_3$-$C_6$ cyclo alkyl; $C_1$-$C_6$ alkyl substituted with hydroxy, $C_1$-$C_6$ alkoxy, mercapto, $C_1$-$C_6$ alkylthio, aryl, heteroaryl, aralkyloxy or aralkylthio; aryl; or heteroaryl; |
| $R^3$ is | $-OR^5$ or $-NR^5R^6$; |
| $R^4$ and $R^9$ are | independently $-(CH_2)_qR^8$; |
| $R^5$ and $R^6$ are | independently selected from the group consisting of hydrogen, $C_1$-$C_6$ alkyl, hydroxy $C_1$-$C_6$ alkyl, $C_1$-$C_6$ alkoxy $C_1$-$C_6$ alkyl and aryl $C_1$-$C_6$ alkyl, or $R^5$ and $R^6$ together with the nitrogen to which they are attached form a 5-7 membered ring; |
| $R^7$ is | phenyl substituted by 1 to 3 substituents selected from the group consisting of $C_1$-$C_6$ alkyl, $C_1$-$C_6$ alkoxy, cycloalkyl, cyano and aminomethyl; |
| $R^8$ is | hydrogen, hydroxy, $C_1$-$C_6$ alkoxy, mercapto, $C_1$-$C_6$ alkylthio, aryl or heteroaryl; |

wherein aryl is a mono-cyclic or fused ring bicyclic carbocyclic aromatic group having 6 to 10 ring members and heteroaryl is a mono-cyclic or fused ring bicyclic aromatic group having 5 to 10 ring members wherein 1-2 ring members are independently nitrogen, oxygen or sulfur, and wherein the carbon ring members of the aryl and heteroaryl groups are substituted by 0-3 substituents selected from $C_1$- $C_6$ alkyl, hydroxy, halo, $C_1$- $C_6$ alkoxy, $C_3$- $C_6$ cyclo alkyl, cyano, aminomethyl, trifluoromethyl, phenyl, phenoxy or phenylthio;

| | |
|---|---|
| n is | 1 or 2; |
| p is | 0 or 1; |
| q is | 0, 1 or 2; and |
| t is | 0 or 1; |

or a pharmaceutically acceptable salt thereof.

**2.** A compound of formula I of claim 1 wherein $R^1$ is phenyl or $C_1$-$C_6$ alkyl substituted phenyl and n is 1.

**3.** A compound of formula I of claim 1 wherein $R^3$ is hydroxy or $C_1$-$C_6$ alkoxy.

**4.** A compound of formula I of claim 1 wherein p is zero.

**5.** A compound of formula I of claim 1 wherein $R^4$ is hydrogen, hydroxy, methoxy, phenyl or benzyl.

**6.** A compound of formula I of claim 1 wherein $R^2$ is hydrogen or thienyl.

**7.** A compound of formula I of claim 1 wherein p and t are each zero, $R^2$ is hydrogen and $R^4$ is hydroxy or methoxy.

**8.** A compound of formula II of claim 1 wherein $R^7$ is $C_1$-$C_6$ alkyl-substituted phenyl and n is 1.

**9.** A compound of formula II of claim 1 wherein $R^2$ is $C_1$-$C_6$ alkylthio $C_1$-$C_6$ alkyl.

**10.** A compound of claim 1 which is:
1,1'-[dithiobis-[2(S)-(2-methylbenzyl)-1-oxo-3,1-propanediyl]]-bis-(S)-isoserine diethyl ester;
1,1'-[dithiobis-[2(S)-(2-methylbenzyl)-1-oxo-3,1-propanediyl]]-bis-(S)-isoserine;
1,1'-[dithiobis[2(S)-(2-methylbenzyl)-1-oxo-3,1-propanediyl]]bis-(S)-methionine diethyl ester;
1,1'-[dithiobis[2(S)-(2-methylbenzyl)-1-oxo-3,1-propanediyl]]bis-(S)-methionine.

**11.** A method for preparing a pharmaceutical composition for treating hypertension, congestive heart failure, edema, renal insufficiency, nephrotoxicity or pain comprising an effective amount of a compound of claim 1, alone or in combination with an atrial nartiuretic factor or an angiotensin converting enzyme inhibitor, in a pharmaceutically acceptable carrier, which method comprises admixing the compound of claim 1 with the pharmaceutically acceptable carrier.

**12.** A method of claim 11 wherein the atrial natriuretic peptide is chosen from α human AP 21, α human AP 28, α human AP 23, α human AP 24, α human AP 25, α human AP 26, α human AP 33, and the corresponding atrial peptides wherein the methionine at position 12 is replaced by isoleucine.

**13.** A method of claim 11 wherein the angiotensin converting enzyme inhibitor is selected from: spirapril, enalapril, ramipril, perindopril, indolapril, lysinopril, quinapril, pentopril, cilazapril, captopril, zofenopril, pivalopril and fosinopril.

**14.** A method for preparing a kit comprising in separate containers in a single package pharmaceutical compositions for use in combination to treat hypertension, congestive heart failure or nephrotoxicity in mammals which method comprises using in one container a pharmaceutical composition comprising a mercapto-acylamino acid disulfide derivative of claim 1 and in a second container a pharmaceutical composition comprising an atrial natriuretic factor.

**15.** A method for preparing a kit comprising in separate containers in a single package pharmaceutical compositions for use in combination to treat hypertension or congestive heart failure in mammals which method comprises using in one container a pharmaceutical composition comprising a mercapto-acylamino acid disulfide derivative of claim 1 and in a second container a pharmaceutical composition comprising an angiotensin converting enzyme inhibitor.

**16.** The use of a compound of claim 1 for the manufacture of a medicament for treating hypertension, congestive heart failure, edema, renal insufficiency, nephrotoxicity or pain.

**17.** A process for the preparation of a compound of formula I or II as defined in claim 1, wherein $R^1$, $R^2$, $R^3$, $R^4$, $R^7$, $R^9$, n, p and t are as defined in claim 1, selected from the following processes A, B, C, and D:
Process A for preparing compounds of formula I comprising amidating an acid of formula IV with an amine of formula V:

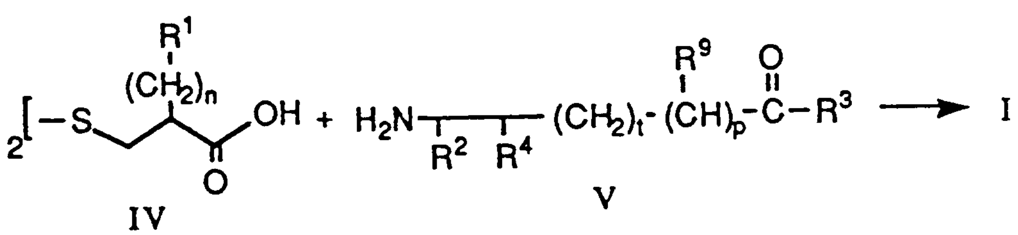

Process B for preparing compounds of formula II comprising amidating an acid of formula VI with an amine of formula VII:

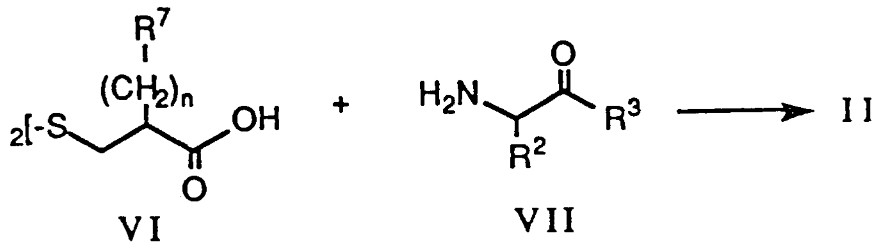

VI VII

Process C for preparing compounds of formula I comprising oxidizing a mercaptoacylamino acid of formula Ia:

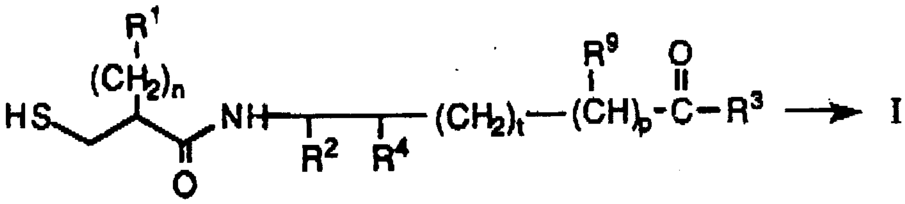

Ia

Process D for preparing compounds of formula II comprising oxidizing a mercaptoacylamino acid of formula IIa:

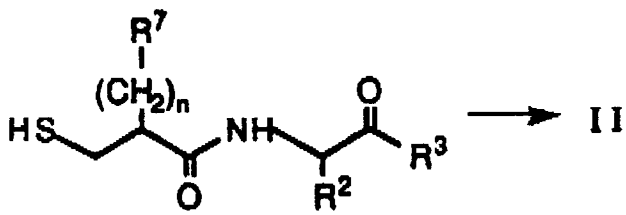

IIa

wherein each process is followed by isolation of the preferred isomer, if desired, and removal of the protecting groups, if necessary, to yield the desired product, and if desired, preparation of a salt thereof.

**Patentansprüche**
**Patentansprüche für folgende Vertragsstaaten : AT, BE, CH, DE, DK, FR, GB, IT, LI, LU, NL, SE**

1. Verbindung mit der Strukturformel

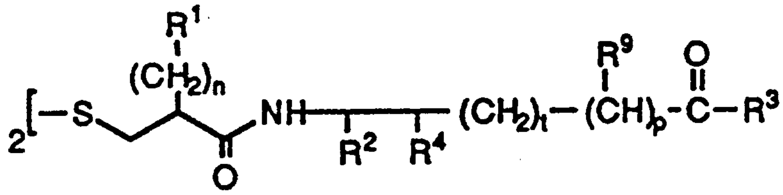

(I)

oder

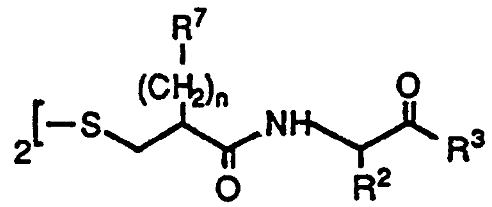

(II)

worin

| | |
|---|---|
| $R^1$ | $C_1$-$C_6$-Alkyl, $C_3$-$C_6$-Cycloalkyl, Aryl oder Heteroaryl ist, |
| $R^2$ | Wasserstoff; $C_1$-$C_6$-Alkyl; $C_3$-$C_6$-Cycloalkyl; mit Hydroxy substituiertes $C_1$-$C_6$-Alkyl, $C_1$-$C_6$-Alkoxy, Mercapto, $C_1$-$C_6$-Alkylthio, Aryl, Heteroaryl, Aralkyloxy oder Aralkylthio; Aryl oder Heteroaryl ist, |
| $R^3$ | $-OR^5$ oder $-NR^5R^6$ ist, |
| $R^4$ und $R^9$ | unabhängig $-(CH_2)_qR^8$ sind, |
| $R^5$ und $R^6$ | unabhängig aus der Gruppe ausgewählt sind, die aus Wasserstoff, $C_1$-$C_6$-Alkyl, Hydroxy-$C_1$-$C_6$-alkyl, $C_1$-$C_6$-Alkoxy-$C_1$-$C_6$-alkyl und Aryl-$C_1$-$C_6$-alkyl besteht, oder $R^5$ und $R^6$ zusammen mit dem Stickstoff, an den sie gebunden sind, einen 5-7gliedrigen Ring bilden, |
| $R^7$ | Phenyl ist, das durch 1 bis 3 Substituenten substituiert ist, die aus der Gruppe ausgewählt sind, welche aus $C_1$-$C_6$-Alkyl, $C_1$-$C_6$-Alkoxy; Cycloalkyl, Cyano und Aminomethyl besteht, |
| $R^8$ | Wasserstoff, Hydroxy, $C_1$-$C_6$-Alkoxy, Mercapto, $C_1$-$C_6$-Alkylthio, Aryl oder Heteroaryl ist, |

worin Aryl eine monocyclische oder mit einem kondensierten Ring versehene, bicyclische, carbocyclische, aromatische Gruppe mit 6 bis 10 Ringgliedern ist und Heteroaryl eine monocyclische oder mit einem kondensierten Ring versehene, bicyclische, aromatische Gruppe mit 5 bis 10 Ringgliedern ist, worin 1-2 Ringglieder unabhängig Stickstoff, Sauerstoff oder Schwefel sind, und worin die Kohlenstoff-Ringglieder der Aryl- und Heteroarylgruppen durch 0-3 Substituenten substituiert sind, die aus $C_1$-$C_6$-Alkyl, Hydroxy, Halogen, $C_1$-$C_6$-Alkoxy, $C_3$-$C_6$-Cycloalkyl, Cyano, Aminomethyl, Trifluormethyl, Phenyl, Phenoxy oder Phenylthio ausgewählt sind,

| | |
|---|---|
| n | 1 oder 2 ist, |
| p | 0 oder 1 ist, |
| q | 0, 1 oder 2 ist und |
| t | 0 oder 1 ist, |

oder ein pharmazeutisch annehmbares Salz derselben.

2. Verbindung der Formel I des Anspruchs 1, worin $R^1$ Phenyl oder durch $C_1$-$C_6$-Alkyl substituiertes Phenyl ist und n 1 ist.

3. Verbindung der Formel I des Anspruchs 1, worin $R^3$ Hydroxy oder $C_1$-$C_6$-Alkoxy ist.

4. Verbindung der Formel I des Anspruchs 1, worin p null ist.

**5.** Verbindung der Formel I des Anspruchs 1, worin $R^4$ Wasserstoff, Hydroxy, Methoxy, Phenyl oder Benzyl ist.

**6.** Verbindung der Formel I des Anspruchs 1, worin $R^2$ Wasserstoff oder Thienyl ist.

**7.** Verbindung der Formel I des Anspruchs 1, worin p und t jeweils null sind, $R^2$ Wasserstoff ist und $R^4$ Hydroxy oder Methoxy ist.

**8.** Verbindung der Formel II des Anspruchs 1, worin $R^7$ durch $C_1$-$C_6$-Alkyl substituiertes Phenyl ist und n 1 ist.

**9.** Verbindung der Formel II des Anspruchs 1, worin $R^2$ $C_1$-$C_6$-Alkylthio-$C_1$-$C_6$-alkyl ist.

**10.** Verbindung des Anspruchs 1, welche
1,1'-[Dithiobis-[2(S)-(2-methylbenzyl)-1-oxo-3,1-propandiyl]]-bis-(S)-isoserindiethylester,
1,1'-[Dithiobis-[2(S)-(2-methylbenzyl)-1-oxo-3,1-propandiyl]]-bis-(S)-isoserin,
1,1'-[Dithiobis-[2(S)-(2-methylbenzyl)-1-oxo-3,1-propandiyl]]-bis-(S)-methionindiethylester,
1,1'-[Dithiobis-[2(S)-(2-methylbenzyl)-1-oxo-3,1-propandiyl]]-bis-(S)-methionin ist.

**11.** Pharmazeutische Zusammensetzung zum Behandeln von Bluthochdruck, Stauungsinsuffizienz, Ödem, Niereninsuffizienz, Nephrotoxizität oder Schmerz, die eine wirksame Menge einer Verbindung des Anspruchs 1 allein oder in Kombination mit einem natriuretischen Faktor oder einem Hemmer des Angiotensin-umwandelnden Enzyms in einem pharmazeutisch annehmbaren Träger enthält.

**12.** Zusammensetzung des Anspruchs 11, worin das natriuretische Peptid aus $\alpha$-Human-AP 21, $\alpha$-Human-AP 28, $\alpha$-Human-AP 23, $\alpha$-Human-AP 24, $\alpha$-Human-AP 25, $\alpha$-Human-AP 26, $\alpha$-Human-AP 33 und den entsprechenden atrialen Peptiden, worin das Methionin in Position 12 durch Isoleucin ersetzt ist, ausgewählt ist.

**13.** Zusammensetzung des Anspruchs 11, worin der Hemmer des Angiotensin-umwandelnden Enzyms aus Spirapril, Enalapril, Ramipril, Perindopril, Indolapril, Lysinopril, Quinapril, Pentopril, Cilazapril, Captopril, Zofenopril, Pivalopril und Fosinopril ausgewählt ist.

**14.** Kit, der in getrennten Behältnissen in einer einzigen Packung pharmazeutische Zusammensetzungen zur kombinierten Verwendung zum Behandeln von Bluthochdruck, Stauungsinsuffizienz oder Nephrotoxizität in Säugern enthält, welche in einem Behältnis eine pharmazeutische Zusammensetzung enthält, die ein Mercaptoacylaminosäuredisulfidderivat des Anspruchs 1 und in einem zweiten Behältnis eine pharmazeutische Zusammensetzung enthält, die einen natriuretischen Faktor umfaßt.

**15.** Kit, der in getrennten Behältnissen in einer einzigen Packung pharmazeutische Zusammensetzungen zur kombinierten Verwendung zum Behandeln von Bluthochdruck oder Stauungsinsuffizienz in Säugern enthält, welche in einem Behältnis eine pharmazeutische Zusammensetzung enthält, die ein Mercaptoacylaminosäuredisulfidderivat des Anspruchs 1 und in einem zweiten Behältnis eine pharmazeutische Zusammensetzung enthält, die einen Hemmer des Angiotensin-umwandelnden Enzyms umfaßt.

**16.** Verfahren zum Herstellen einer pharmazeutischen Zusammensetzung, welches das Mischen einer Verbindung des Anspruchs 1 mit einem pharmazeutisch annehmbaren Träger umfaßt.

**17.** Verwendung einer Verbindung des Anspruchs 1 zur Herstellung eines Arzneimittels zum Behandeln von Bluthochdruck, Stauungsinsuffizienz, Ödem, Niereninsuffizienz, Nephrotoxizität oder Schmerz.

**18.** Verfahren zur Herstellung einer Verbindung der in Anspruch 1 definierten Formel I oder II, worin $R^1$, $R^2$, $R^3$, $R^4$, $R^7$, $R^9$, n, p und t wie in Anspruch 1 definiert sind, welches aus den folgenden Verfahren A, B, C und D ausgewählt ist:

Verfahren A zum Herstellen von Verbindungen der Formel I, welches das Amidieren einer Säure der Formel IV mit einem Amin der Formel V umfaßt:

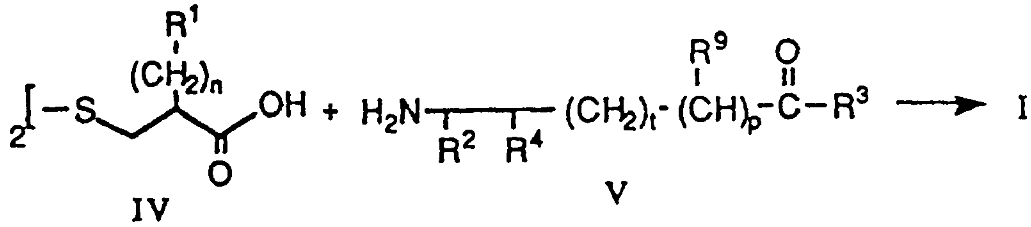

IV V

Verfahren B zum Herstellen von Verbindungen der Formel II, welches das Amidieren einer Säure der Formel VI mit einem Amin der Formel VII umfaßt:

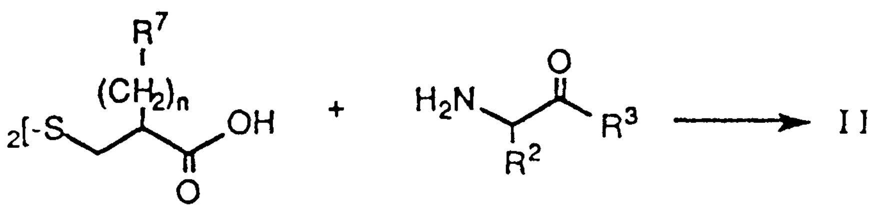

VI VII

Verfahren C zum Herstellen von Verbindungen der Formel I, welches das Oxidieren einer Mercaptoacylaminosäure der Formel Ia umfaßt:

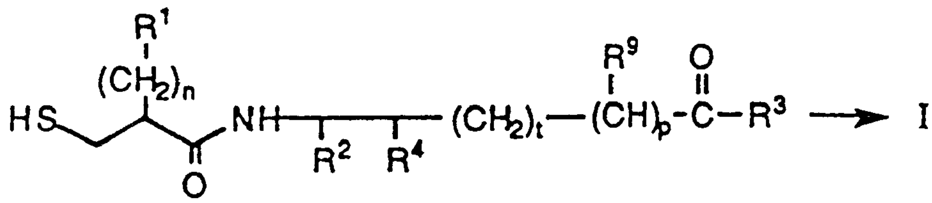

Ia

Verfahren D zum Herstellen von Verbindungen der Formel II, welches das Oxidieren einer Mercaptoacylaminosäure der Formel IIa umfaßt:

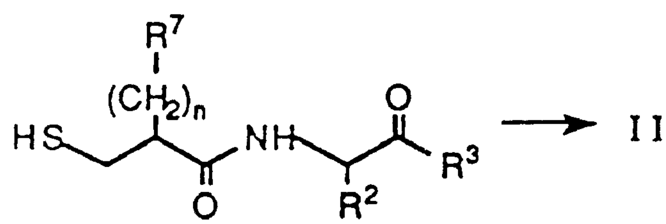

IIa

wobei jedes Verfahren gewünschtenfalls von der Isolierung des bevorzugten Isomers und nötigenfalls der Entfernung der Schutzgruppen unter Liefern des gewünschten Produkts und gewünschtenfalls der Herstellung eines Salzes derselben gefolgt wird.

**Patentansprüche für folgenden Vertragsstaat : ES**

**1.** Verfahren zur Herstellung einer Verbindung mit der Strukturformel

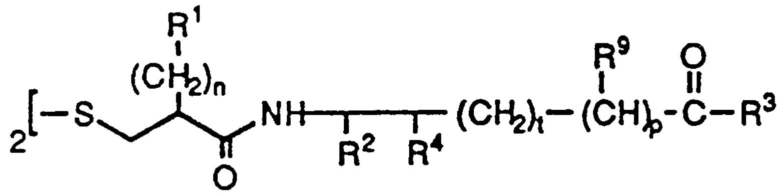

(I)

oder

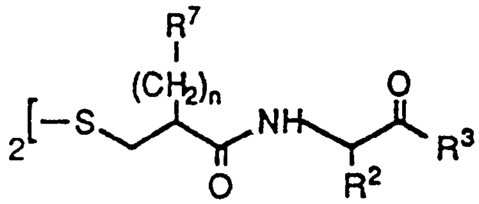

(II)

worin

$R^1$ $C_1$-$C_6$-Alkyl, $C_3$-$C_6$-Cycloalkyl, Aryl oder Heteroaryl ist,

$R^2$ Wasserstoff; $C_1$-$C_6$-Alkyl; $C_3$-$C_6$-Cycloalkyl; mit Hydroxy substituiertes $C_1$-$C_6$-Alkyl, $C_1$-$C_6$-Alkoxy, Mercapto, $C_1$-$C_6$-Alkylthio, Aryl, Heteroaryl, Aralkyloxy oder Aralkylthio; Aryl oder Heteroaryl ist,

$R^3$ -$OR^5$ oder -$NR^5R^6$ ist,

$R^4$ und $R^9$ unabhängig -$(CH_2)_qR^8$ sind,

$R^5$ und $R^6$ unabhängig aus der Gruppe ausgewählt sind, die aus Wasserstoff, $C_1$-$C_6$-Alkyl, Hydroxy-$C_1$-$C_6$-alkyl, $C_1$-$C_6$-Alkoxy-$C_1$-$C_6$-alkyl und Aryl-$C_1$-$C_6$-alkyl besteht, oder $R^5$ und $R^6$ zusammen mit dem Stickstoff, an den sie gebunden sind, einen 5-7gliedrigen Ring bilden,

$R^7$ Phenyl ist, das durch 1 bis 3 Substituenten substituiert ist, die aus der Gruppe ausgewählt sind, welche aus $C_1$-$C_6$-Alkyl, $C_1$-$C_6$-Alkoxy; Cycloalkyl, Cyano und Aminomethyl besteht,

$R^8$ Wasserstoff, Hydroxy, $C_1$-$C_6$-Alkoxy, Mercapto, $C_1$-$C_6$-Alkylthio, Aryl oder Heteroaryl ist,

worin Aryl eine monocyclische oder mit einem kondensierten Ring versehene, bicyclische, carbocyclische, aromatische Gruppe mit 6 bis 10 Ringgliedern ist und Heteroaryl eine monocyclische oder mit einem kondensierten Ring versehene, bicyclische, aromatische Gruppe mit 5 bis 10 Ringgliedern ist, worin 1-2 Ringglieder unabhängig Stickstoff, Sauerstoff oder Schwefel sind, und worin die Kohlenstoff-Ringglieder der Aryl- und Heteroarylgruppen durch 0-3 Substituenten substituiert sind, die aus $C_1$-$C_6$-Alkyl, Hydroxy, Halogen, $C_1$-$C_6$-Alkoxy, $C_3$-$C_6$-Cycloalkyl, Cyano, Aminomethyl, Trifluormethyl, Phenyl, Phenoxy oder Phenylthio ausgewählt sind,

n 1 oder 2 ist,

p 0 oder 1 ist,

q 0, 1 oder 2 ist und

t 0 oder 1 ist,

oder eines pharmazeutisch annehmbaren Salzes derselben, wobei das Verfahren aus den folgenden Verfahren A, B, C und D ausgewählt ist:

Verfahren A zum Herstellen von Verbindungen der Formel I, welches das Amidieren einer Säure der Formel IV mit einem Amin der Formel V umfaßt:

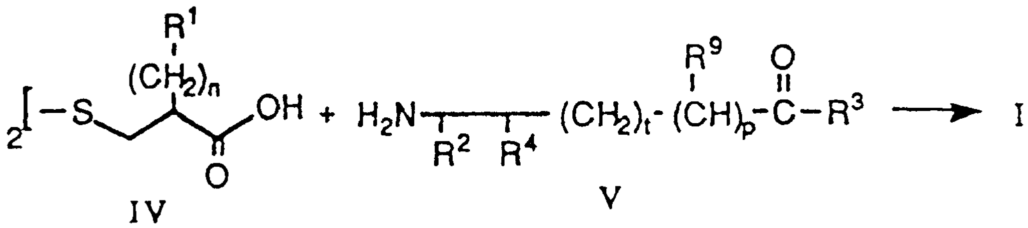

IV V

Verfahren B zum Herstellen von Verbindungen der Formel II, welches das Amidieren einer Säure der Formel VI mit einem Amin der Formel VII umfaßt:

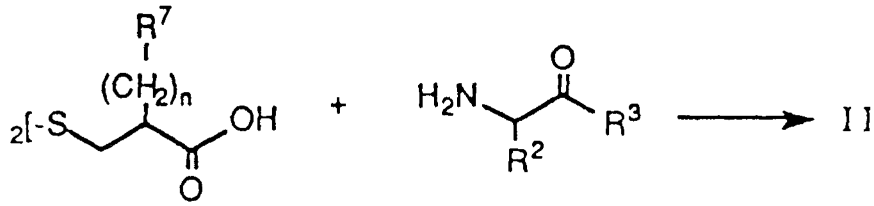

VI VII

Verfahren C zum Herstellen von Verbindungen der Formel I, welches das Oxidieren einer Mercaptoacylaminosäure der Formel Ia umfaßt:

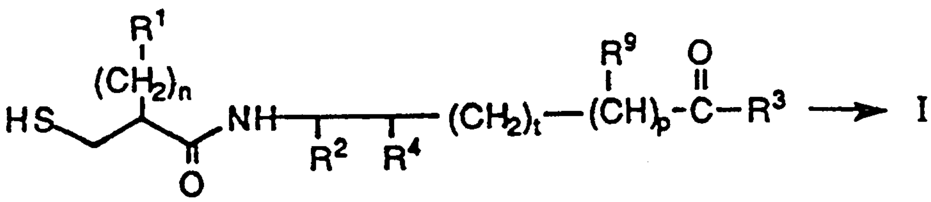

Ia

Verfahren D zum Herstellen von Verbindungen der Formel II, welches das Oxidieren einer Mercaptoacylaminosäure der Formel IIa umfaßt:

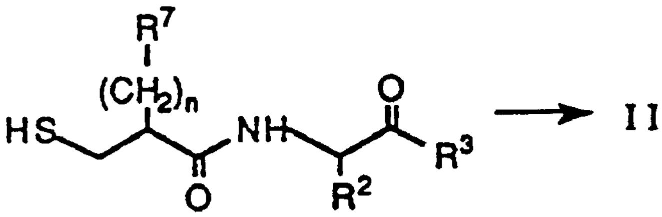

IIa

wobei jedes Verfahren gewünschtenfalls von der Isolierung des bevorzugten Isomers und nötigenfalls der Entfernung der Schutzgruppen unter Liefern des gewünschten Produkts und gewünschtenfalls der Herstellung eines Salzes derselben gefolgt wird.

**2.** Verfahren des Anspruchs 1, wobei in einer Verbindung der Formel I $R^1$ Phenyl oder durch $C_1$-$C_6$-Alkyl substituiertes Phenyl ist und n 1 ist.

**3.** Verfahren des Anspruchs 1, wobei in einer Verbindung der Formel I $R^3$ Hydroxy oder $C_1$-$C_6$-Alkoxy ist.

**4.** Verfahren des Anspruchs 1, wobei in einer Verbindung der Formel I p null ist.

5. Verfahren des Anspruchs 1, wobei in einer Verbindung der Formel I $R^4$ Wasserstoff, Hydroxy, Methoxy, Phenyl oder Benzyl ist.

6. Verfahren des Anspruchs 1, wobei in einer Verbindung der Formel I $R^2$ Wasserstoff oder Thienyl ist.

7. Verfahren des Anspruchs 1, wobei in einer Verbindung der Formel I p und t jeweils null sind, $R^2$ Wasserstoff ist und $R^4$ Hydroxy oder Methoxy ist.

8. Verfahren des Anspruchs 1, wobei in einer Verbindung der Formel II $R^7$ durch $C_1$-$C_6$-Alkyl substituiertes Phenyl ist und n 1 ist.

9. Verfahren des Anspruchs 1, wobei in einer Verbindung der Formel II $R^2$ $C_1$-$C_6$-Alkylthio-$C_1$-$C_6$-alkyl ist.

10. Verfahren des Anspruchs 1, wobei die hergestellte Verbindung
1,1'-[Dithiobis-[2(S)-(2-methylbenzyl)-1-oxo-3,1-propandiyl]]-bis-(S)-isoserindiethylester,
1,1'-[Dithiobis-[2(S)-(2-methylbenzyl)-1-oxo-3,1-propandiyl]]-bis-(S)-isoserin,
1,1'-[Dithiobis-[2(S)-(2-methylbenzyl)-1-oxo-3,1-propandiyl]]-bis-(S)-methionindiethylester,
1,1'-[Dithiobis-[2(S)-(2-methylbenzyl)-1-oxo-3,1-propandiyl]]-bis-(S)-methionin ist.

11. Verfahren zum Herstellen einer pharmazeutischen Zusammensetzung zum Behandeln von Bluthochdruck, Stauungsinsuffizienz, Ödem, Niereninsuffizienz, Nephrotoxizität oder Schmerz, die eine wirksame Menge einer Verbindung hergestellt entspechend des Anspruchs 1 allein oder in Kombination mit einem natriuretischen Faktor oder einem Hemmer des Angiotensin-umwandelnden Enzyms in einem pharmazeutisch annehmbaren Träger enthält, wobei das Verfahren das Mischen der gemäß Anspruch 1 hergestellten Verbindung mit dem pharmazeutisch annehmbaren Träger umfaßt.

12. Verfahren des Anspruchs 11, wobei das natriuretische Peptid aus $\alpha$-Human-AP 21, $\alpha$-Human-AP 28, $\alpha$-Human-AP 23, $\alpha$-Human-AP 24, $\alpha$-Human-AP 25, $\alpha$-Human-AP 26, $\alpha$-Human-AP 33 und den entsprechenden atrialen Peptiden, worin das Methionin in Position 12 durch Isoleucin ersetzt ist, ausgewählt ist.

13. Verfahren des Anspruchs 11, wobei der Hemmer des Angiotensin-umwandelnden Enzyms aus Spirapril, Enalapril, Ramipril, Perindopril, Indolapril, Lysinopril, Quinapril, Pentopril, Cilazapril, Captopril, Zofenopril, Pivalopril und Fosinopril ausgewählt ist.

14. Verfahren zum Herstellen eines Kits, der in getrennten Behältnissen in einer einzigen Packung pharmazeutische Zusammensetzungen zur kombinierten Verwendung zum Behandeln von Bluthochdruck, Stauungsinsuffizienz oder Nephrotoxizität in Säugern enthält, wobei das Verfahren das Verwenden einer pharmazeutischen Zusammensetzung, die ein gemäß Anspruch 1 hergestelltes Mercaptoacylaminosäuredisulfidderivat enthält, in einem Behältnis und einer pharmazeutischen Zusammensetzung, die einen natriuretischen Faktor enthält, in einem zweiten Behältnis umfaßt.

15. Verfahren zum Herstellen eines Kits, der in getrennten Behältnissen in einer einzigen Packung pharmazeutische Zusammensetzungen zur kombinierten Verwendung zum Behandeln von Bluthochdruck, Stauungsinsuffizienz oder Nephrotoxizität in Säugern enthält, wobei das Verfahren das Verwenden einer pharmazeutischen Zusammensetzung, die ein gemäß Anspruch 1 hergestelltes Mercaptoacylaminosäuredisulfidderivat enthält, in einem Behältnis und einer pharmazeutischen Zusammensetzung, die einen Hemmer des Angiotensin-umwandelnden Enzyms enthält, in einem zweiten Behältnis umfaßt.

16. Verfahren der Verwendung einer gemäß Anspruch 1 hergestellten Verbindung zum Behandeln von Bluthochdruck, Stauungsinsuffizienz, Ödem, Niereninsuffizienz, Nephrotoxizität oder Schmerz.

**Patentansprüche für folgenden Vertragsstaat : GR**

1. Verbindung mit der Strukturformel

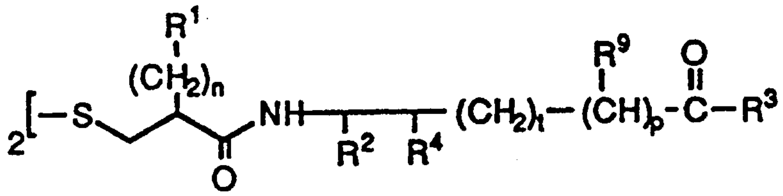

(I)

oder

(II)

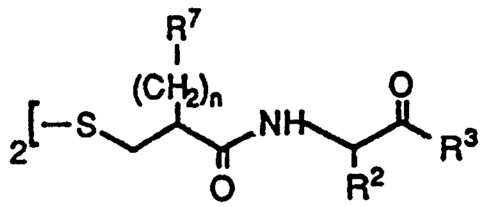

worin

$R^1$ $C_1$-$C_6$-Alkyl, $C_3$-$C_6$-Cycloalkyl, Aryl oder Heteroaryl ist,

$R^2$ Wasserstoff; $C_1$-$C_6$-Alkyl; $C_3$-$C_6$-Cycloalkyl; mit Hydroxy substituiertes $C_1$-$C_6$-Alkyl, $C_1$-$C_6$-Alkoxy, Mercapto, $C_1$-$C_6$-Alkylthio, Aryl, Heteroaryl, Aralkyloxy oder Aralkylthio; Aryl oder Heteroaryl ist,

$R^3$ -$OR^5$ oder -$NR^5R^6$ ist,

$R^4$ und $R^9$ unabhängig -$(CH_2)_qR^8$ sind,

$R^5$ und $R^6$ unabhängig aus der Gruppe ausgewählt sind, die aus Wasserstoff, $C_1$-$C_6$-Alkyl, Hydroxy-$C_1$-$C_6$-alkyl, $C_1$-$C_6$-Alkoxy-$C_1$-$C_6$-alkyl und Aryl-$C_1$-$C_6$-alkyl besteht, oder $R^5$ und $R^6$ zusammen mit dem Stickstoff, an den sie gebunden sind, einen 5-7gliedrigen Ring bilden,

$R^7$ Phenyl ist, das durch 1 bis 3 Substituenten substituiert ist, die aus der Gruppe ausgewählt sind, welche aus $C_1$-$C_6$-Alkyl, $C_1$-$C_6$-Alkoxy; Cycloalkyl, Cyano und Aminomethyl besteht,

$R^8$ Wasserstoff, Hydroxy, $C_1$-$C_6$-Alkoxy, Mercapto, $C_1$-$C_6$-Alkylthio, Aryl oder Heteroaryl ist,

worin Aryl eine monocyclische oder mit einem kondensierten Ring versehene, bicyclische, carbocyclische, aromatische Gruppe mit 6 bis 10 Ringgliedern ist und Heteroaryl eine monocyclische oder mit einem kondensierten Ring versehene, bicyclische, aromatische Gruppe mit 5 bis 10 Ringgliedern ist, worin 1-2 Ringglieder unabhängig Stickstoff, Sauerstoff oder Schwefel sind, und worin die Kohlenstoff-Ringglieder der Aryl- und Heteroarylgruppen durch 0-3 Substituenten substituiert sind, die aus $C_1$-$C_6$-Alkyl, Hydroxy, Halogen, $C_1$-$C_6$-Alkoxy, $C_3$-$C_6$-Cycloalkyl, Cyano, Aminomethyl, Trifluormethyl, Phenyl, Phenoxy oder Phenylthio ausgewählt sind,

n 1 oder 2 ist,

p 0 oder 1 ist,

q 0, 1 oder 2 ist und

t 0 oder 1 ist,

oder ein pharmazeutisch annehmbares Salz derselben.

2. Verbindung der Formel I des Anspruchs 1, worin $R^1$ Phenyl oder durch $C_1$-$C_6$-Alkyl substituiertes Phenyl ist und n 1 ist.

3. Verbindung der Formel I des Anspruchs 1, worin $R^3$ Hydroxy oder $C_1$-$C_6$-Alkoxy ist.

4. Verbindung der Formel I des Anspruchs 1, worin p null ist.

5. Verbindung der Formel I des Anspruchs 1, worin $R^4$ Wasserstoff, Hydroxy, Methoxy, Phenyl oder Benzyl ist.

6. Verbindung der Formel I des Anspruchs 1, worin $R^2$ Wasserstoff oder Thienyl ist.

7. Verbindung der Formel I des Anspruchs 1, worin p und t jeweils null sind, $R^2$ Wasserstoff ist und $R^4$ Hydroxy oder Methoxy ist.

8. Verbindung der Formel II des Anspruchs 1, worin $R^7$ durch $C_1$-$C_6$-Alkyl substituiertes Phenyl ist und n 1 ist.

9. Verbindung der Formel II des Anspruchs 1, worin $R^2$ $C_1$-$C_6$-Alkylthio-$C_1$-$C_6$-alkyl ist.

10. Verbindung des Anspruchs 1, welche
1,1'-[Dithiobis-[2(S)-(2-methylbenzyl)-1-oxo-3,1-propandiyl]]-bis-(S)-isoserindiethylester,
1,1'-[Dithiobis-[2(S)-(2-methylbenzyl)-1-oxo-3,1-propandiyl]]-bis-(S)-isoserin,
1,1'-[Dithiobis-[2(S)-(2-methylbenzyl)-1-oxo-3,1-propandiyl]]-bis-(S)-methionindiethylester,
1,1'-[Dithiobis-[2(S)-(2-methylbenzyl)-1-oxo-3,1-propandiyl]]-bis-(S)-methionin ist.

11. Verfahren zum Herstellen einer pharmazeutischen Zusammensetzung zum Behandeln von Bluthochdruck, Stauungsinsuffizienz, Ödem, Niereninsuffizienz, Nephrotoxizität oder Schmerz, die eine wirksame Menge einer Verbindung des Anspruchs 1 allein oder in Kombination mit einem natriuretischen Faktor oder einem Hemmer des Angiotensin-umwandelnden Enzyms in einem pharmazeutisch annehmbaren Träger enthält, wobei das Verfahren das Mischen der Verbindung des Anspruchs 1 mit dem pharmazeutisch annehmbaren Träger umfaßt.

12. Verfahren des Anspruchs 11, wobei das natriuretische Peptid aus $\alpha$-Human-AP 21, $\alpha$-Human-AP 28, $\alpha$-Human-AP 23, $\alpha$-Human-AP 24, $\alpha$-Human-AP 25, $\alpha$-Human-AP 26, $\alpha$-Human-AP 33 und den entsprechenden atrialen Peptiden, worin das Methionin in Position 12 durch Isoleucin ersetzt ist, ausgewählt ist.

13. Verfahren des Anspruchs 11, wobei der Hemmer des Angiotensin-umwandelnden Enzyms aus Spirapril, Enalapril, Ramipril, Perindopril, Indolapril, Lysinopril, Quinapril, Pentopril, Cilazapril, Captopril, Zofenopril, Pivalopril und Fosinopril ausgewählt ist.

14. Verfahren zum Herstellen eines Kits, der in getrennten Behältnissen in einer einzigen Packung pharmazeutische Zusammensetzungen zur kombinierten Verwendung zum Behandeln von Bluthochdruck, Stauungsinsuffizienz oder Nephrotoxizität in Säugern enthält, wobei das Verfahren das Verwenden einer pharmazeutischen Zusammensetzung, die ein gemäß Anspruch 1 hergestelltes Mercaptoacylaminosäuredisulfidderivat enthält, in einem Behältnis und einer pharmazeutischen Zusammensetzung, die einen natriuretischen Faktor enthält, in einem zweiten Behältnis umfaßt.

15. Verfahren zum Herstellen eines Kits, der in getrennten Behältnissen in einer einzigen Packung pharmazeutische Zusammensetzungen zur kombinierten Verwendung zum Behandeln von Bluthochdruck, Stauungsinsuffizienz oder Nephrotoxizität in Säugern enthält, wobei das Verfahren das Verwenden einer pharmazeutischen Zusammensetzung, die ein gemäß Anspruch 1 hergestelltes Mercaptoacylaminosäuredisulfidderivat enthält, in einem Behältnis und einer pharmazeutischen Zusammensetzung, die einen Hemmer des Angiotensin-umwandelnden Enzyms enthält, in einem zweiten Behältnis umfaßt.

16. Verwendung einer Verbindung des Anspruchs 1 zur Herstellung eines Arzneimittels zum Behandeln von Bluthochdruck, Stauungsinsuffizienz, Ödem, Niereninsuffizienz, Nephrotoxizität oder Schmerz.

17. Verfahren zur Herstellung einer Verbindung der in Anspruch 1 definierten Formel I oder II, worin $R^1$, $R^2$, $R^3$, $R^4$, $R^7$, $R^9$, n, p und t wie in Anspruch 1 definiert sind, welches aus den folgenden Verfahren A, B, C und D ausgewählt ist:

Verfahren A zum Herstellen von Verbindungen der Formel I, welches das Amidieren einer Säure der Formel IV mit einem Amin der Formel V umfaßt:

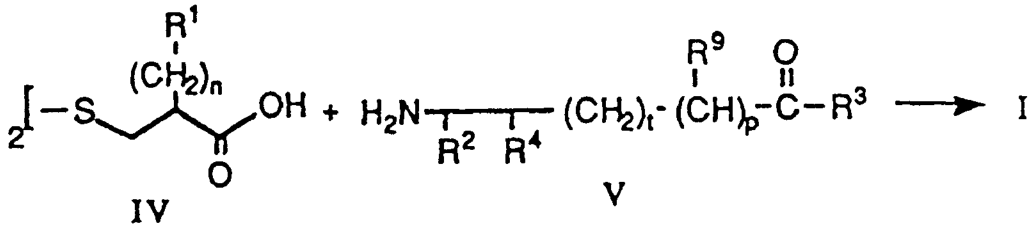

IV V

Verfahren B zum Herstellen von Verbindungen der Formel II, welches das Amidieren einer Säure der Formel VI mit einem Amin der Formel VII umfaßt:

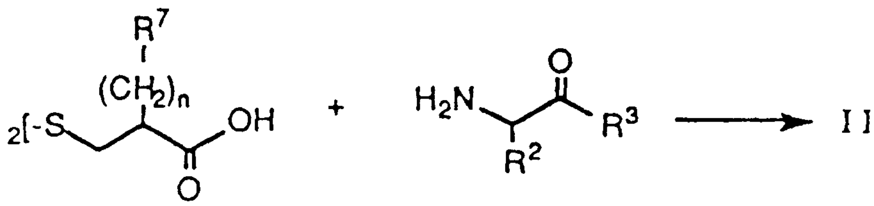

VI VII

Verfahren C zum Herstellen von Verbindungen der Formel I, welches das Oxidieren einer Mercaptoacylaminosäure der Formel Ia umfaßt:

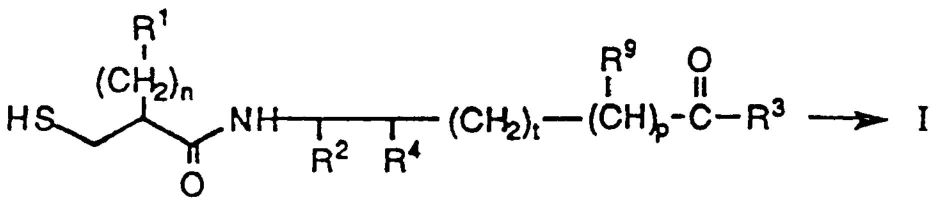

Ia

Verfahren D zum Herstellen von Verbindungen der Formel II, welches das Oxidieren einer Mercaptoacylaminosäure der Formel IIa umfaßt:

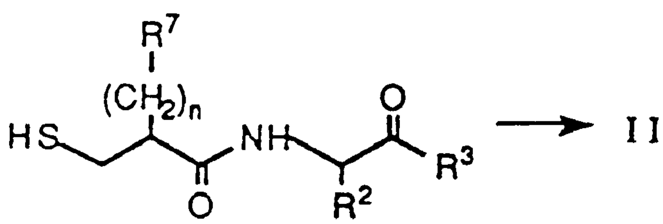

IIa

wobei jedes Verfahren gewünschtenfalls von der Isolierung des bevorzugten Isomers und nötigenfalls der Entfernung der Schutzgruppen unter Liefern des gewünschten Produkts und gewünschtenfalls der Herstellung eines Salzes derselben gefolgt wird.

## Revendications

**Revendications pour les Etats contractants suivants : AT, BE, CH, DE, DK, FR, GB, IT, LI, LU, NL, SE**

1. Composé ayant la formule structurelle

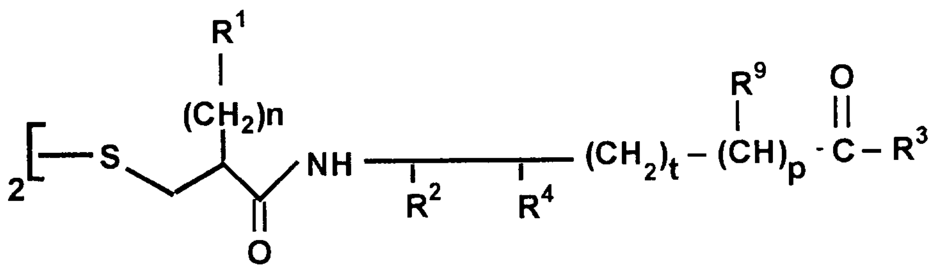

I

ou

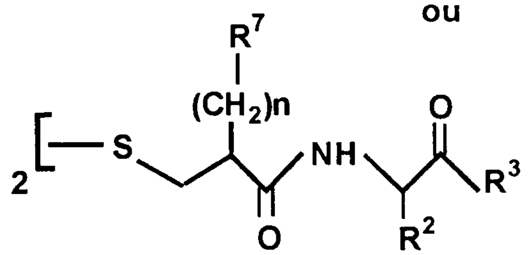

II

où

$R^1$ est un alkyle en $C_1$-$C_6$, un cycloalkyle en $C_3$-$C_6$, un aryle ou un hétéroaryle;

$R^2$ est un hydrogène; un alkyle en $C_1$-$C_6$; un cycloalkyle en $C_3$-$C_6$; un alkyle en $C_1$-$C_6$ substitué avec un hydroxy, un alcoxy en $C_1$-$C_6$, un mercapto, un alkylthio en $C_1$-$C_6$, un aryle, un hétéroaryle, un aralkyloxy ou un aralkylthio; un aryle; ou un hétéroaryle;

$R^3$ est $-OR^5$ ou $-NR^5R^6$;

$R^4$ et $R^9$ sont indépendamment $-(CH_2)_qR^8$;

$R^4$ et $R^5$ sont indépendamment choisis dans le groupe consistant d'un hydrogène, d'un alkyle en $C_1$-$C_6$, d'un hydroxy-alkyle $C_1$-$C_6$, d'un alcoxy en $C_1$-$C_6$ alkyle en $C_1$-$C_6$ et d'un arylalkyle en $C_1$-$C_6$, ou $R^5$ et $R^6$ ensemble avec l'azote auquel ils sont attachés forment un cycle à 5-7 membres;

$R^7$ est un phényle substitué par 1 à 3 substituants choisis dans le groupe consistant d'un alkyle en $C_1$-$C_6$, d'un alcoxy en $C_1$-$C_6$, d'un cycloalkyle, d'un cyano et d'un aminométhyle;

$R^8$ est un hydrogène, un hydroxy, un alcoxy en $C_1$-$C_6$, un mercapto, un alkylthio en $C_1$-$C_6$, un aryle ou un hétéroaryle;

où l'aryle est un groupe aromatique carbocyclique bicyclique à cycles fusionnés ou monocyclique ayant 6 à 10 membres et l'hétéroaryle est un groupe aromatique bicyclique à cycles fusionnés ou monocyclique ayant 5 à 10 membres par cycle ou 1-2 membres par cycle sont indépendamment l'azote, l'oxygène ou le soufre, et où les membres carbone des cycles des groupes aryle et hétéroaryle sont substitués par 0-3 substituants choisis parmi un alkyle en $C_1$-$C_6$, un hydroxy, un halo, un alcoxy en $C_1$-$C_6$, un cycloalkyle en $C_3$-$C_6$, un cyano, un aminométhyle, un trifluorométhyle, un phényle, un phénoxy ou un phénylthio;

n est égal à 1 ou 2;

p est égal à 0 ou 1;

q est égal à 0, 1 ou 2; et

t est égal à 0 ou 1;

ou un sel pharmaceutiquement acceptable de celui-ci.

2. Composé de formule I selon la revendication 1 où $R^1$ est un phényle ou un phényle substitué par un alkyle en $C_1$-$C_6$ et n est égal à 1.

3. Composé de formule I selon la revendication 1 où $R^3$ est un hydroxy ou un alcoxy en $C_1$-$C_6$.

4. Composé de formule I de la revendication 1 où p est égal à zéro.

5. Composé de formule I selon la revendication 1 où $R^4$ est un hydrogène, un hydroxy, un méthoxy, un phényle ou un benzyle.

6. Composé de formule I selon la revendication 1 où $R^2$ est un hydrogène ou un thiényle.

7. Composé de formule I de la revendication 1 où p et t sont chacun égaux à zéro, $R^2$ est un hydrogène et $R^4$ est un hydroxy ou un méthoxy.

8. Composé de formule II selon la revendication 1 où $R^7$ est un phényle substitué par un alkyle en $C_1$-$C_6$ et n est égal à 1.

9. Composé de formule II selon la revendication 1 où $R^2$ est un alkylthio en $C_1$-$C_6$, un alkyle en $C_1$-$C_6$.

10. Composé selon la revendication 1 qui est :
    le diéthyl ester de la 1,1'-[dithiobis-[2(S)-(2-méthylbenzyl)-1-oxo-3,1-propanediyl]]-bis(S)-isosérine;
    la 1,1'-[dithiobis-[2(S)-(2-méthylbenzyl)-1-oxo-3,1-propanédiyl]]-bis-(S)-isosérine;
    le diéthyl ester de la 1,1'-[dithiobis[2(S)-(2-méthylbenzyl)-1-oxo-3,1-propanediyl]]bis-(S)-méthionine.
    la 1,1'-[dithiobis-[2(S)-(2-méthylbenzyl)-1-oxo-3,1-propanediyl]]-bis-(S)-méthionine.

11. Composition pharmaceutique pour traiter l'hypertension, l'insuffisance cardiaque congestive, l'oedème, l'insuffisance rénale, la néphrotoxicité ou la douleur comprenant une quantité efficace d'un composé selon la revendication 1, seul ou en combinaison avec un facteur natriurétique atrial ou un inhibiteur d'enzyme convertissant l'angiotensine, dans un porteur pharmaceutiquement acceptable.

12. Composition selon la revendication 11 où le peptide natriurétique atrial est choisi parmi l'AP21 $\alpha$ humain, l'AP28 $\alpha$ humain, l'AP23 $\alpha$ humain, l'AP24 $\alpha$ humain, l'AP25 $\alpha$ humain, l'AP26 $\alpha$ humain, l'AP33 $\alpha$ humain, et les peptides atriaux correspondants où la méthionine en position 12 est remplacée par l'isoleucine.

13. Composition selon la revendication 11, où l'inhibiteur d'enzyme convertissant l'angiotensine est choisi parmi : le spiraprile, l'énalaprile, le ramiprile, le périndoprile, l'indolaprile, le lysinoprile, le quinaprile, le pentoprile, le cilazaprile, le captoprile, le zofénoprile, le pivaloprile et le fosinoprile.

14. Kit comprenant dans des conteneurs séparés dans un emballage unique des compositions pharmaceutiques pour utilisation combinée pour traiter l'hypertension, la défaillance cardiaque congestive ou la néphrotoxicité chez des mammifères qui comprend dans un conteneur une composition pharmaceutique comprenant un dérivé disulfure d'acide mercaptoacylaminé selon la revendication 1 et dans un second conteneur une composition pharmaceutique comprenant un facteur natriurétique atrial.

15. Kit comprenant dans des conteneurs séparés dans un emballage unique des compositions pharmaceutiques pour utilisation en combinaison pour traiter l'hypertension ou la défaillance cardiaque congestive chez les mammifères qui comprend dans un conteneur une composition pharmaceutique comprenant un dérivé disulfure d'acide mercapto-acylaminé selon la revendication 1 et dans un second conteneur une composition pharmaceutique comprenant un inhibiteur d'enzyme convertissant l'angiotensine.

16. Méthode pour préparer une composition pharmaceutique comprenant le mélange de composés selon la revendication 1 avec un porteur pharmaceutiquement acceptable.

17. Utilisation d'un composé selon la revendication 1 pour la fabrication d'un médicament pour traiter l'hypertension, la défaillance cardiaque congestive, l'oedème, l'insuffisance rénale, la néphrotoxicité ou la douleur.

18. Procédé pour la préparation d'un composé de formule 1 ou 2 tel que défini à la revendication 1, où $R^1$, $R^2$, $R^3$, $R^4$, $R^7$, $R^9$, n, p et t sont tels que définis en revendication 1, choisi parmi les procédés A, B, C, et D :

    le procédé A pour préparer des composés de formule I comprenant l'amidation d'un acide de formule IV avec une amine de formule V :

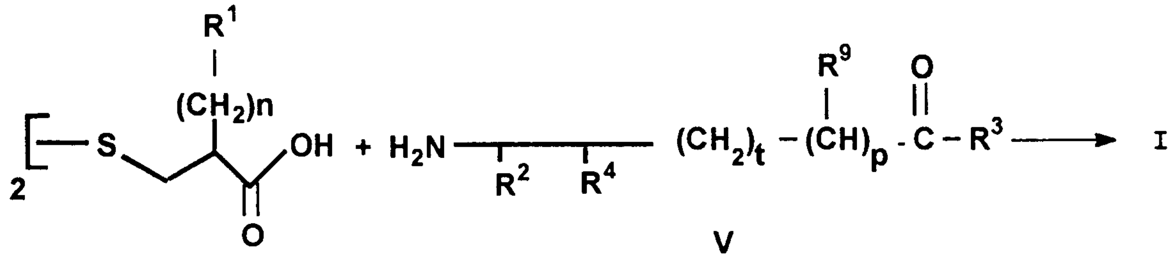

V

IV

le procédé B pour préparer les composés de formule II comprenant l'amidation d'un acide de formule VI avec une amine de formule VII :

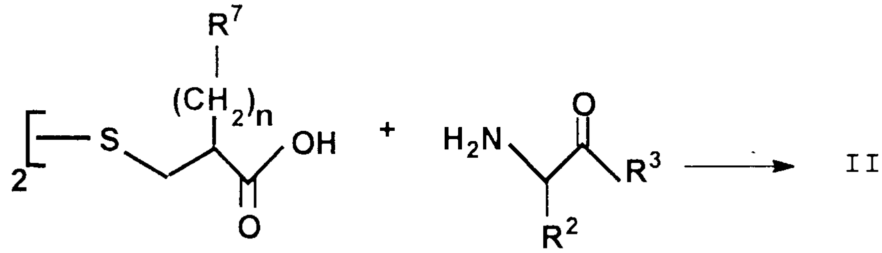

VI VII

le procédé C pour préparer des composés de formule I comprenant l'oxydation d'un acide mercaptoacylaminé de formule Ia :

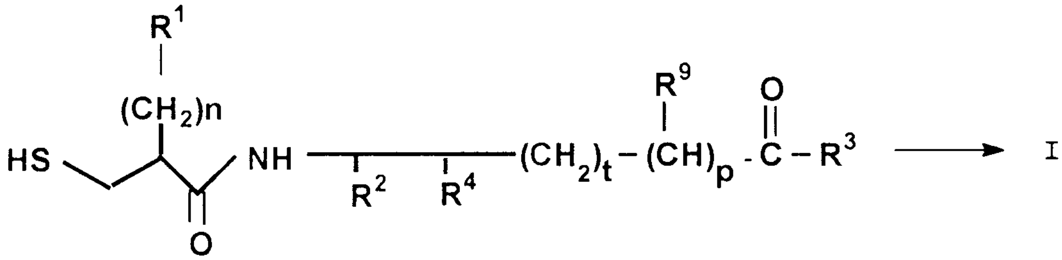

le procédé D pour préparer des composés de formule II comprenant l'oxydation d'un acide mercaptoacylaminé de formule IIa :

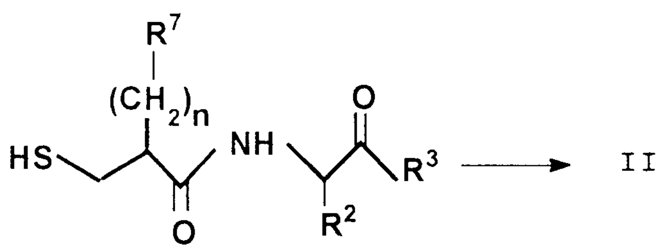

IIa

où chaque procédé est suivi par l'isolement de l'isomère préféré, si désiré, et l'élimination des groupes protecteurs, si nécessaire, pour donner le produit désiré, et si désiré, la préparation d'un sel de celui-ci.

**Revendications pour l'Etat contractant suivant : ES**

1. Procédé pour la préparation d'un composé ayant la formule structurelle

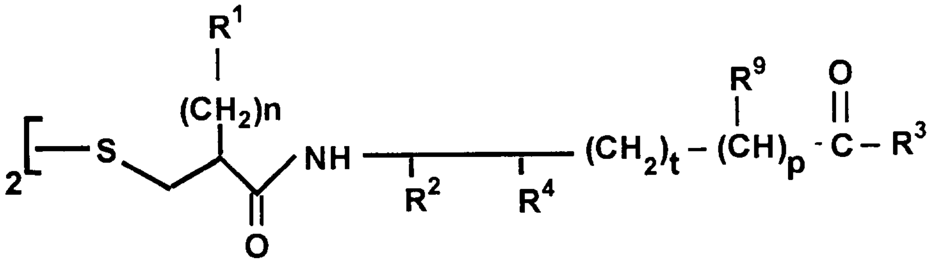

I

ou

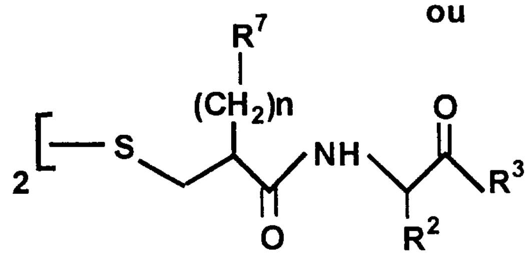

II

où
$R^1$ est un alkyle en $C_1$-$C_6$, un cycloalkyle en $C_3$-$C_6$, un aryle ou un hétéroaryle;
$R^2$ est un hydrogène; un alkyle en $C_1$-$C_6$; un cycloalkyle en $C_3$-$C_6$; un alkyle en $C_1$-$C_6$ substitué avec un hydroxy, un alcoxy en $C_1$-$C_6$, un mercapto, un alkylthio en $C_1$-$C_6$, un aryle, un hétéroaryle, un aralkyloxy ou un aralkylthio; un aryle; ou un hétéroaryle;
$R^3$ est $-OR^5$ ou $-NR^5R^6$;
$R^4$ et $R^9$ sont indépendamment $-(CH_2)_qR^8$;
$R^4$ et $R^5$ sont indépendamment choisis dans le groupe consistant d'un hydrogène, d'un alkyle en $C_1$-$C_6$, d'un hydroxy-alkyle $C_1$-$C_6$, d'un alcoxy en $C_1$-$C_6$ alkyle en $C_1$-$C_6$ et d'un arylalkyle en $C_1$-$C_6$, ou $R^5$ et $R^6$ ensemble avec l'azote auquel ils sont attachés forment un cycle à 5-7 membres;
$R^7$ est un phényle substitué par 1 à 3 substituants choisis dans le groupe consistant d'un alkyle en $C_1$-$C_6$, d'un alcoxy en $C_1$-$C_6$, d'un cycloalkyle, d'un cyano et d'un amino-méthyle;
$R^8$ est un hydrogène, un hydroxy, un alcoxy en $C_1$-$C_6$, un mercapto, un alkylthio en $C_1$-$C_6$, un aryle ou un hétéroaryle;
où l'aryle est un groupe aromatique carbocyclique bicyclique à cycles fusionnés ou monocyclique ayant 6 à 10 membres et l'hétéroaryle est un groupe aromatique bicyclique à cycles fusionnés ou monocyclique ayant 5 à 10 membres par cycle ou 1-2 membres par cycle sont indépendamment l'azote, l'oxygène ou le soufre, et où les membres carbone des cycles des groupes aryle et hétéroaryle sont substitués par 0-3 substituants choisis parmi un alkyle en $C_1$-$C_6$, un hydroxy, un halo, un alcoxy en $C_1$-$C_6$, un cycloalkyle en $C_3$-$C_6$, un cyano, un aminométhyle, un trifluorométhyle, un phényle, un phénoxy ou un phénylthio;
n est égal à 1 ou 2;
p est égal à 0 ou 1;
q est égal à 0, 1 ou 2; et
t est égal à 0 ou 1;
ou un sel pharmaceutiquement acceptable de celui-ci, lequel procédé étant choisi parmi les procédés A, B, C, et
D :
le procédé A pour préparer des composés de formule I comprenant l'amidation d'un acide de formule IV avec une amine de formule V :

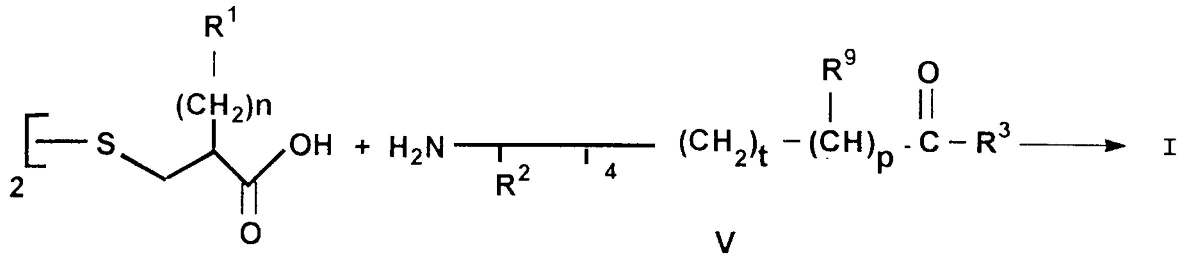

IV V

le procédé B pour préparer les composés de formule II comprenant l'amidation d'un acide de formule VI avec une amine de formule VII :

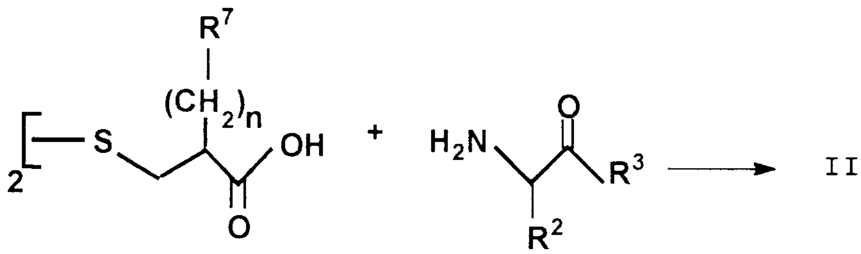

VI VII

le procédé C pour préparer des composés de formule I comprenant l'oxydation d'un acide mercaptoacylaminé de formule Ia :

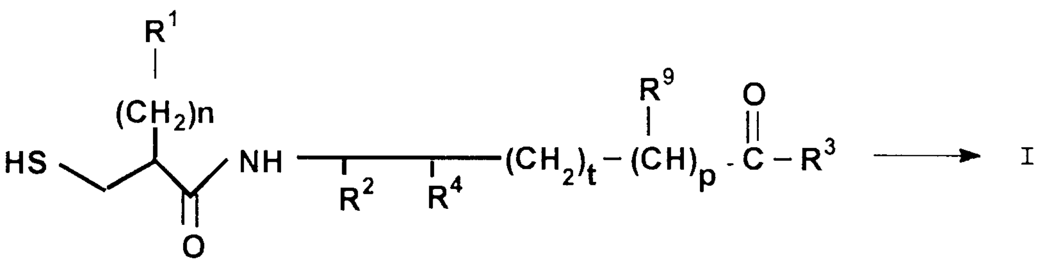

Ia

le procédé D pour préparer des composés de formule II comprenant l'oxydation d'un acide mercaptoacylaminé de formule IIa :

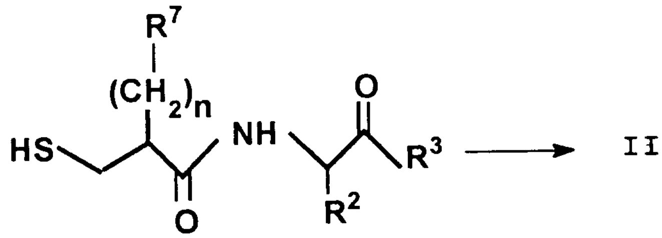

IIa

où chaque procédé est suivi par l'isolement de l'isomère préféré, si désiré, et l'élimination des groupes

protecteurs, si nécessaire, pour donner le produit désiré, et si désiré, la préparation d'un sel de celui-ci.

2. Procédé selon la revendication 1 où dans un composé de formule I $R^1$ est un phényle ou un phényle substitué par un alkyle en $C_1$-$C_6$ et n est égal à 1.

3. Procédé selon la revendication 1 où dans un composé de formule I $R^3$ est un hydroxy ou un alcoxy en $C_1$-$C_6$.

4. Procédé selon la revendication 1 où dans un composé de formule I où p est égal à zéro.

5. Procédé selon la revendication 1 où dans un composé de formule I $R^4$ est un hydrogène, un hydroxy, un méthoxy, un phényle ou un benzyle.

6. Procédé selon la revendication 1 où dans un composé de formule I $R^2$ est un hydrogène ou un thiényle.

7. Procédé selon la revendication 1 où dans un composé de formule I p et t sont chacun égaux à zéro, $R^2$ est un hydrogène et $R^4$ est un hydroxy ou un méthoxy.

8. Procédé selon la revendication 1 où dans un composé de formule II $R^7$ est un phényle substitué par un alkyle en $C_1$-$C_6$ et n est égal à 1.

9. Procédé selon la revendication 1 où dans un composé de formule II $R^2$ est un alkylthio en $C_1$-$C_6$, en alkyle en $C_1$-$C_6$.

10. Procédé selon la revendication 1 où le composé produit est :
le diéthyl ester de la 1,1'-[dithiobis-[2(S)-(2-méthylbenzyl)-1-oxo-3,1-propanediyle]]-bis(S)-isosérine;
la 1,1'-[dithiobis-[2(S)-(2-méthylbenzyl)-1-oxo-3,1-propanédiyl]]-bis-(S)-isosérine;
le diéthyl ester de la 1,1'-[dithiobis[2(S)-(2-méthylbenzyl)-1-oxo-3,1-propanediyl]]bis-(S)-méthionine.
la 1,1'-[dithiobis-[2(S)-(2-méthylbenzyl)-1-oxo-3,1-propanediyl]]-bis-(S)-méthionine.

11. Méthode de préparation d'une composition pharmaceutique pour traiter l'hypertension, l'insuffisance cardiaque congestive, l'oedème, l'insuffisance rénale, la néphrotoxicité ou la douleur comprenant une quantité efficace d'un composé préparé selon la revendication 1, seul ou en combinaison avec un facteur natriurétique atrial ou un inhibiteur d'enzyme convertissant l'angiotensine, dans un porteur pharmaceutiquement acceptable.

12. Méthode selon la revendication 11 où le peptide natriurétique atrial est choisi parmi l'AP21 $\alpha$ humain, l'AP28 $\alpha$ humain, l'AP23 $\alpha$ humain, l'AP24 $\alpha$ humain, l'AP25 $\alpha$ humain, l'AP26 $\alpha$ humain, l'AP33 $\alpha$ humain, et les peptides atriaux correspondants ou la méthionine en position 12 est remplacée par l'isoleucine.

13. Méthode selon la revendication 11, où l'inhibiteur d'enzyme convertissant l'angiotensine est choisi parmi : le spiraprile, l'énalaprile, le ramiprile, le périndoprile, l'indolaprile, le lysinoprile, le quinaprile, le pentoprile, le cilazaprile, le captoprile, le zofénoprile, le pivaloprile et le fosinoprile.

14. Méthode pour préparer un kit comprenant dans des conteneurs séparés dans un emballage unique des compositions pharmaceutiques pour utilisation combinée pour traiter l'hypertension, la défaillance cardiaque congestive ou la néphrotoxicité chez des mammifères laquelle méthode comprend l'utilisation de, dans un conteneur, une composition pharmaceutique comprenant un dérivé disulfure d'acide mercaptoacylaminé préparé selon la revendication 1 et, dans un second conteneur une composition pharmaceutique comprenant un facteur natriurétique atrial.

15. Méthode pour préparer un kit comprenant dans des conteneurs séparés dans un emballage unique des compositions pharmaceutiques pour utilisation en combinaison pour traiter l'hypertension ou la défaillance cardiaque congestive chez les mammifères laquelle méthode comprend l'utilisation de, dans un conteneur, une composition pharmaceutique comprenant un dérivé disulfure d'acide mercaptoacylaminé préparé selon la revendication 1 et de, dans un second conteneur une composition pharmaceutique

comprenant un inhibiteur d'enzyme convertissant l'angiotensine.

**16.** Méthode d'utilisation d'un composé préparé selon la revendication 1 pour la fabrication d'un médicament pour traiter l'hypertension, la défaillance cardiaque congestive, l'oedème, l'insuffisance rénale, la néphrotoxicité ou la douleur.

**Revendications pour l'Etat contractant suivant : GR**

**1.** Composé ayant la formule structurelle

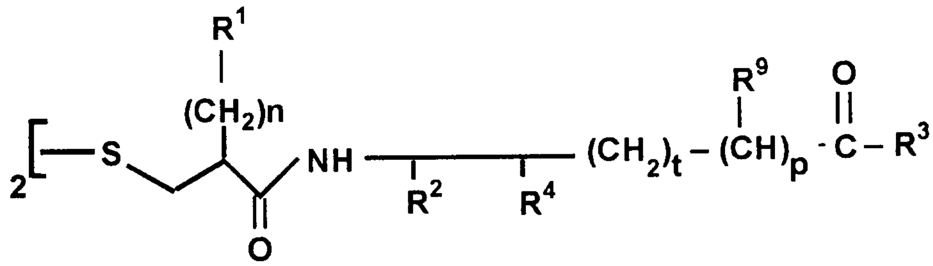

I

ou

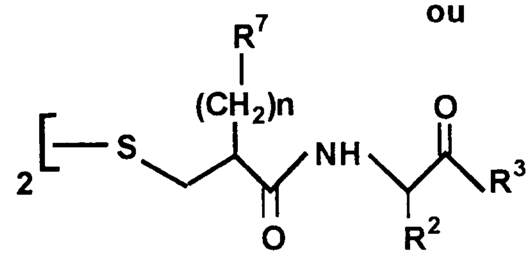

II

où

$R^1$ est un alkyle en $C_1$-$C_6$, un cycloalkyle en $C_3$-$C_6$, un aryle ou un hétéroaryle;
$R^2$ est un hydrogène; un alkyle en $C_1$-$C_6$; un cycloalkyle en $C_3$-$C_6$; un alkyle en $C_1$-$C_6$ substitué avec un hydroxy, un alcoxy en $C_1$-$C_6$, un mercapto, un alkylthio en $C_1$-$C_6$, un aryle, un hétéroaryle, un aralkyloxy ou un aralkylthio; un aryle; ou un hétéroaryle;
$R^3$ est -$OR^5$ ou -$NR^5R^6$;
$R^4$ et $R^9$ sont indépendamment -$(CH_2)_qR^8$;
$R^4$ et $R^5$ sont indépendamment choisis dans le groupe consistant d'un hydrogène, d'un alkyle en $C_1$-$C_6$, d'un hydroxy-alkyle $C_1$-$C_6$, d'un alcoxy en $C_1$-$C_6$ alkyle en $C_1$-$C_6$ et d'un arylalkyle en $C_1$-$C_6$, ou $R^5$ et $R^6$ ensemble avec l'azote auquel ils sont attachés forment un cycle à 5-7 membres;
$R^7$ est un phényle substitué par 1 à 3 substituants choisis dans le groupe consistant d'un alkyle en $C_1$-$C_6$, d'un alcoxy en $C_1$-$C_6$, d'un cycloalkyle, d'un cyano et d'un amino-méthyle;
$R^8$ est un hydrogène, un hydroxy, un alcoxy en $C_1$-$C_6$, un mercapto, un alkylthio en $C_1$-$C_6$, un aryle ou un hétéroaryle;
où l'aryle est un groupe aromatique carbocyclique bicyclique à cycles fusionnés ou monocyclique ayant 6 à 10 membres et l'hétéroaryle est un groupe aromatique bicyclique à cycles fusionnés ou monocyclique ayant 5 à 10 membres par cycle ou 1-2 membres par cycle sont indépendamment l'azote, l'oxygène ou le soufre, et où les membres carbone des cycles des groupes aryle et hétéroaryle sont substitués par 0-3 substituants choisis parmi un alkyle en $C_1$-$C_6$, un hydroxy, un halo, un alcoxy en $C_1$-$C_6$, un cycloalkyle en $C_3$-$C_6$, un cyano, un aminométhyle, un trifluorométhyle, un phényle, un phénoxy ou un phénylthio;
n est égal à 1 ou 2;
p est égal à 0 ou 1;
q est égal à 0, 1 ou 2; et
t est égal à 0 ou 1;
ou un sel pharmaceutiquement acceptable de celui-ci.

**2.** Composé de formule I selon la revendication 1 où $R^1$ est un phényle ou un phényle substitué par un alkyle en $C_1$-$C_6$ et n est égal à 1.

**3.** Composé de formule I selon la revendication 1 où $R^3$ est un hydroxy ou un alcoxy en $C_1$-$C_6$.

**4.** Composé de formule I de la revendication 1 où p est égal à zéro.

**5.** Composé de formule I selon la revendication 1 où $R^4$ est un hydrogène, un hydroxy, un méthoxy, un phényle ou un benzyle.

**6.** Composé de formule I selon la revendication 1 où $R^2$ est un hydrogène ou un thiényle.

**7.** Composé de formule I de la revendication 1 où p et t sont chacun égaux à zéro, $R^2$ est un hydrogène et $R^4$ est un hydroxy ou un méthoxy.

**8.** Composé de formule II selon la revendication 1 où $R^7$ est un phényle substitué par un alkyle en $C_1$-$C_6$ et n est égal à 1.

**9.** Composé de formule II selon la revendication 1 où $R^2$ est un alkylthio en $C_1$-$C_6$, en alkyle en $C_1$-$C_6$.

**10.** Composé selon la revendication 1 qui est :
le diéthyl ester de la 1,1'-[dithiobis-[2(S)-(2-méthylbenzyl)-1-oxo-3,1-propanediyle]]-bis(S)-isosérine;
la 1,1'-[dithiobis-[2(S)-(2-méthylbenzyl)-1-oxo-3,1-propanédiyl]]-bis-(S)-isosérine;
le diéthyl ester de la 1,1'-[dithiobis[2(S)-(2-méthylbenzyl)-1-oxo-3,1-propanediyl]]bis-(S)-méthionine.
la 1,1'-[dithiobis-[2(S)-(2-méthylbenzyl)-1-oxo-3,1-propanediyl]]-bis-(S)-méthionine.

**11.** Méthode pour préparer une composition pharmaceutique pour traiter l'hypertension, l'insuffisance cardiaque congestive, l'oedème, l'insuffisance rénale, la néphrotoxicité ou la douleur comprenant une quantité efficace d'un composé selon la revendication 1, seul ou en combinaison avec un facteur natriurétique atrial ou un inhibiteur d'enzyme convertissant l'angiotensine, dans un porteur pharmaceutiquement acceptable, laquelle méthode comprend le mélange du composé selon la revendication 1 avec le porteur pharmaceutiquement acceptable.

**12.** Méthode selon la revendication 11 où le peptide natriurétique atrial est choisi parmi l'AP21 $\alpha$ humain, l'AP28 $\alpha$ humain, l'AP23 $\alpha$ humain, l'AP24 $\alpha$ humain, l'AP25 $\alpha$ humain, l'AP26 $\alpha$ humain, l'AP33 $\alpha$ humain, et les peptides atriaux correspondants où la méthionine en position 12 est remplacée par l'isoleucine.

**13.** Méthode selon la revendication 11, où l'inhibiteur d'enzyme convertissant l'angiotensine est choisi parmi : le spiraprile, l'énalaprile, le ramiprile, le périndoprile, l'indolaprile, le lysinoprile, le quinaprile, le pentoprile, le cilazaprile, le captoprile, le zofénoprile, le pivaloprile et le fosinoprile.

**14.** Méthode pour préparer un kit comprenant dans des conteneurs séparés dans un emballage unique des compositions pharmaceutiques pour utilisation combinée pour traiter l'hypertension, la défaillance cardiaque congestive ou la néphrotoxicité chez des mammifères laquelle méthode comprend l'utilisation de, dans un conteneur une composition pharmaceutique comprenant un dérivé disulfure d'acide mercaptoacylaminé selon la revendication 1 et de, dans un second conteneur une composition pharmaceutique comprenant un facteur natriurétique atrial.

**15.** Méthode pour préparer un kit comprenant dans des conteneurs séparés dans un emballage unique des compositions pharmaceutiques pour utilisation en combinaison pour traiter l'hypertension ou la défaillance cardiaque congestive chez les mammifères qui comprend dans un conteneur une composition pharmaceutique comprenant un dérivé disulfure d'acide mercapto-acylaminé selon la revendication 1 et dans un second conteneur une composition pharmaceutique comprenant un inhibiteur d'enzyme convertissant l'angiotensine.

**16.** Utilisation d'un composé selon la revendication 1 pour la fabrication d'un médicament pour traiter l'hypertension, la défaillance cardiaque congestive, l'oedème, l'insuffisance rénale, la néphrotoxicité ou la douleur.

**17.** Procédé pour la préparation d'un composé de formule 1 ou 2 tel que défini à la revendication 1, où $R^1$, $R^2$, $R^3$, $R^4$, $R^7$, $R^9$, n, p et t sont tels que définis en revendication 1, choisi parmi les procédés A, B, C, et D :

le procédé A pour préparer des composés de formule I comprenant l'amidation d'un acide de formule IV avec une amine de formule V :

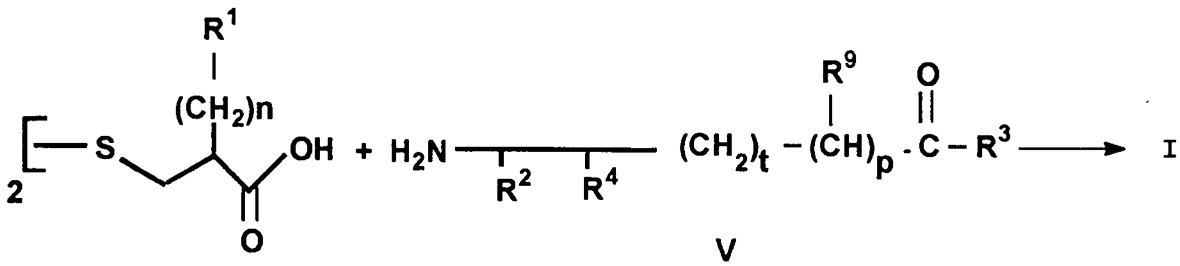

IV V I

le procédé B pour préparer les composés de formule II comprenant l'amidation d'un acide de formule VI avec une amine de formule VII :

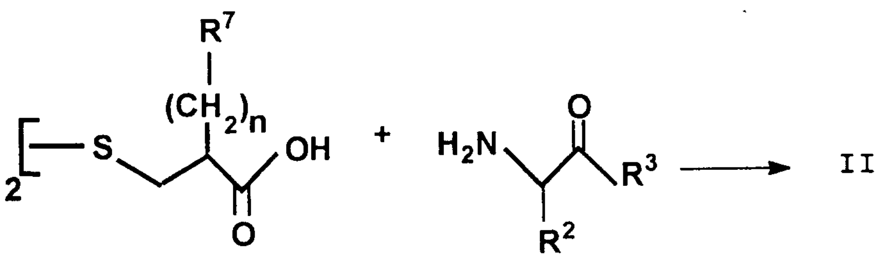

VI VII II

le procédé C pour préparer des composés de formule I comprenant l'oxydation d'un acide mercaptoacylaminé de formule Ia :

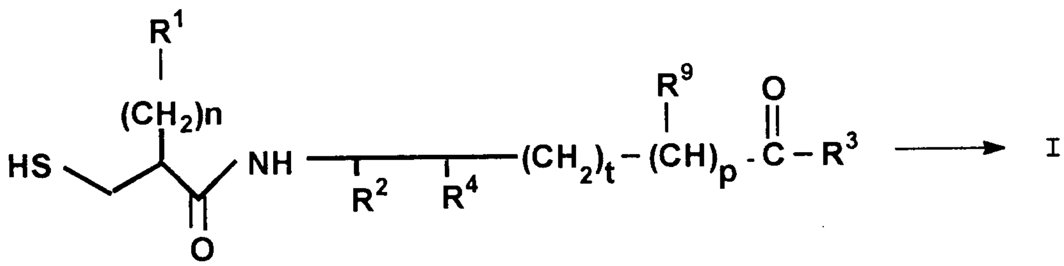

Ia I

le procédé D pour préparer des composés de formule II comprenant l'oxydation d'un acide mercaptoacylaminé de formule IIa :

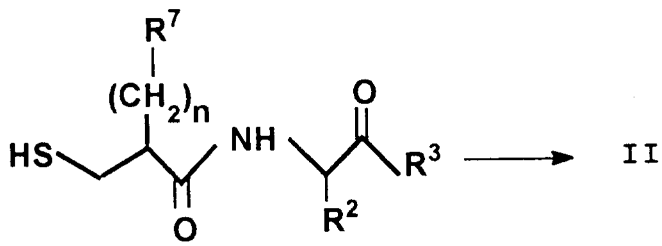

IIa

où chaque procédé est suivi par l'isolement de l'isomère préféré, si désiré, et l'élimination des groupes protecteurs, si nécessaire, pour donner le produit désiré, et si désiré, la préparation d'un sel de celui-ci.